# EUROPEAN PATENT APPLICATION

(11) **EP 3 951 402 A1**
(43) Date of publication of application: **09.02.2022**
(21) Application number: 20784884.7
(22) Date of filing: 01.04.2020
(51) Int. Cl.: G01N 37/00, C12M 1/00, C12M 1/34, G01N 35/08

(54) **FLOW CHANNEL DEVICE AND INSPECTION SYSTEM**

(30) Priority: 02.04.2019 JP 2019070591
(71) Applicant: H.U. Group Research Institute G.K., Tokyo 192-0031 (JP)
(72) Inventor: TONOMURA, Wataru, Hachioji-shi Tokyo 192-0031 (JP); MATSUNO, Tatsuki, Hachioji-shi Tokyo 192-0031 (JP); KOTAKA, Takeyuki, Hachioji-shi Tokyo 192-0031 (JP)
(74) Representative: Laine IP Oy
(86) International application number: PCT/JP2020/015007
(87) International publication number: WO 2020/204067

(57) **Abstract**

To provide a flow path device and a test system of which the ease of use can be improved.

A flow path device includes a flow path 50. The flow path 50 includes a plurality of inlet portion side first flow paths 61 connected to an inlet portion 30; a plurality of inlet portion side second flow paths 62 connected to the plurality of respective inlet portion side first flow paths 61; and a plurality of reaction chamber portions 63 connected to the plurality of respective inlet portion side second flow paths 62. The plurality of inlet portion side first flow paths 61 are configured such that when a positive pressure is applied to a liquid in the plurality of inlet portion side first flow paths 61, the amounts of the liquid flowing out from the plurality of inlet portion side first flow paths 61 to the respective reaction chamber portions 63 are substantially the same. The plurality of inlet portion side second flow paths 62 are configured such that when the magnitude of the positive pressure applied to the liquid in the plurality of inlet portion side first flow paths 61 is less than a threshold value, the outflow of the liquid from the inlet portion side first flow paths 61 to the reaction chamber portions 63 is restricted, and when the magnitude of the positive pressure is the threshold value or greater, the outflow of the liquid is allowed.

## Description

### Technical Field

The present invention relates to a flow path device and a test system.

### Background Art

In the related art, a flow path device has been proposed which is to be used for a nucleic acid amplification test among biological component tests. For example, Patent Document 1 discloses a flow path device including a flat plate-shaped lid substrate; a flat plate-shaped flow path substrate joined to the lid substrate; and a plurality of flow paths provided between the lid substrate and the flow path substrate and including respective supply ports and discharge ports. In addition, the plurality of flow paths each are formed in a linear shape and disposed apart from each other (namely, disposed so as not to be connected to each other). With such a configuration, nucleic acid amplification tests for different analysis items can be performed in the plurality of flow paths, and multi-item analysis can be performed using one flow path device.

### Citation List

### Patent Document

Patent Document 1: JP-A-2016-200504

### Summary of the Invention

### Technical Problem

However, in the flow path device of the related art, as described above, since the plurality of flow paths are disposed so as not to be connected to each other, even when a plurality of reactions are performed on the same liquid, it is necessary to individually inject the predetermined liquid into the plurality of flow paths via the respective supply ports. Therefore, it takes labor and time to carry out work for containing the liquid, so that there is room for improvement from the viewpoint of ease of use of the flow path device.

An object of the invention is to solve the above problem in the related art, and provide a flow path device and a test system of which the ease of use can be improved.

### Means to Solve Problem

In order to solve the above mentioned problem and achieve the purpose, a biological component test flow path device of claim 1 is a flow path device that contains a liquid containing a test object to be used for a biochemical test, a hematology test, a nucleic acid test, or an immunological test, the device comprising: a main body portion; a flow path provided inside the main body portion; an inlet portion which is provided so as to be exposed to an outside of the main body portion and is connected to the flow path, and through which the liquid flows into the flow path; and an outlet portion which is provided so as to be exposed to the outside of the main body portion and is connected to the flow path, and through which air in the flow path flows out to the outside, wherein the flow path includes a plurality of first flow paths connected to the inlet portion and having hydrophilicity, a plurality of second flow paths each of which is connected to one of the plurality of first flow paths, and a plurality of reaction chamber portions each of which is used for reaction of the liquid and is connected to one of the plurality of second flow paths, the plurality of first flow paths are configured such that when a positive pressure or negative pressure is applied to the liquid in the plurality of first flow paths by a predetermined method, amounts of the liquid flowing out from the plurality of first flow paths toward the corresponding reaction chamber portions are substantially the same, and the plurality of second flow paths are configured such that when a magnitude of the positive pressure or negative pressure applied to the liquid in the plurality of first flow paths is less than a threshold value, an outflow of the liquid from the first flow path, which is connected to each of the plurality of second flow paths, to the corresponding reaction chamber portion is restricted, and when the magnitude of the applied positive pressure or negative pressure is the threshold value or greater, an outflow of the liquid is allowed.

The flow path device of claim 2 according to the flow path device of claim 1, wherein the inlet portion is a dispensing opening through which the liquid is dispensed to the plurality of first flow paths, and is an application opening through which a positive pressure is applied to the liquid in the plurality of first flow paths.

The flow path device of claim 3 according to the flow path device of claim 1 or 2, wherein hydraulic equivalent diameters of the plurality of first flow paths are set to be the same, and lengths of the plurality of first flow paths are set to be the same.

The flow path device of claim 4 according to the flow path device of any one of claims 1 to 3, wherein the plurality of first flow paths are formed in a meandering shape.

The flow path device of claim 5 according to the flow path device of any one of claims 1 to 4, wherein at least a part of each of inner walls of the plurality of first flow paths has hydrophilicity over an entire length of the first flow path.

The flow path device of claim 6 according to the flow path device of any one of claims 1 to 5, wherein the number of the reaction chamber portions connected to each of the plurality of second flow paths is set to a power of 2.

The flow path device of claim 7 according to the flow path device of any one of claims 1 to 6, wherein in each of inner walls of the plurality of second flow paths, at least a portion connected to the first flow path and a portion connected to the reaction chamber portion have hydrophobicity.

The flow path device of claim 8 according to the flow path device of any one of claims 1 to 7, wherein at least a part of each of inner walls of the plurality of reaction chamber portions has hydrophilicity.

A test system of claim 9 comprising: the flow path device according to any one of claims 1 to 8.

### Effect of Invention

According to the flow path device of claim 1 and the test system of claim 9, since the flow path includes a plurality of first flow paths connected to the inlet portion and having hydrophilicity, a plurality of second flow paths each of which is connected to one of the plurality of first flow paths, and a plurality of reaction chamber portions each of which is used for reaction of the liquid and is connected to one of the plurality of second flow paths, the plurality of first flow paths are configured such that when a positive pressure or negative pressure is applied to the liquid in the plurality of first flow paths by a predetermined method, amounts of the liquid flowing out from the plurality of first flow paths toward the corresponding reaction chamber portions are substantially the same, and the plurality of second flow paths are configured such that when a magnitude of the positive pressure or negative pressure applied to the liquid in the plurality of first flow paths is less than a threshold value, an outflow of the liquid from the first flow path, which is connected to each of the plurality of second flow paths, to the corresponding reaction chamber portion is restricted, and when the magnitude of the applied positive pressure or negative pressure is the threshold value or greater, an outflow of the liquid is allowed, when the liquid is primary dispensed to the plurality of first flow paths via the inlet portion, and a positive pressure or negative pressure is applied to the primary dispensed liquid, the liquid can secondary flow out to the plurality of reaction chamber portions, and the labor of work for dispensing the liquid can be further reduced as compared to the technique of the related art (technique in which the supply port and the discharge port are provided in each of the plurality of flow paths). Particularly, since the plurality of first flow paths can be configured such that the amounts of the liquid flowing out from the plurality of first flow paths are substantially the same, the liquid can evenly flow out to the plurality of reaction chamber portions, and quantitative dispensing to the plurality of reaction chamber portions can be realized. In addition, since the plurality of second flow paths can be configured such that when the magnitude of the positive pressure or negative pressure applied to the liquid in the plurality of first flow paths is less than the threshold value, the outflow of the liquid is restricted, and when the magnitude of the applied positive pressure or negative pressure is the threshold value or greater, the outflow of the liquid is allowed, regardless of the number of the first flow paths that are installed, quantitative dispensing to the plurality of reaction chamber portions can be reliably performed. For the above reasons, the ease of use of the flow path device can be improved.

According to the flow path device of claim 2, since the inlet portion is a dispensing opening through which the liquid is dispensed to the plurality of first flow paths, and is an application opening through which a positive pressure is applied to the liquid in the plurality of first flow paths, the dispensing of the liquid or the application of a positive pressure can be performed via the inlet portion, and the labor of work for dispensing the liquid and work for applying a positive pressure can be further reduced as compared to when the dispensing opening and the application opening are individually provided.

According to the flow path device of claim 3, since hydraulic equivalent diameters of the plurality of first flow paths are set to be the same, and lengths of the plurality of first flow paths are set to be the same, the amounts of the liquid flowing out from the plurality of first flow paths can be made substantially the same, and when positive pressures or negative pressures are applied to the liquid in the plurality of first flow paths, the magnitudes of the applied positive pressures or negative pressures can be made substantially the same, so that quantitative dispensing to the plurality of reaction chamber portions is easily realized.

According to the flow path device of claim 4, since the plurality of first flow paths are formed in a meandering shape, it is possible to make the first flow paths compact and increase the containing capacity, and the ease of use of the flow path device is easily improved.

According to the flow path device of claim 5, since at least a part of each of inner walls of the plurality of first flow paths has hydrophilicity over an entire length of the first flow path, the plurality of first flow paths can reliably have hydrophilicity, and the function of the plurality of first flow paths can be reliably exhibited.

According to the flow path device of claim 6, since the number of the reaction chamber portions connected to each of the plurality of second flow paths is set to a power of 2, the liquid can evenly flow into the reaction chamber portions connected to each of the plurality of second flow paths, and quantitative dispensing to the plurality of reaction chamber portions is easily realized.

According to the flow path device of claim 7, since in each of inner walls of the plurality of second flow paths, at least a portion connected to the first flow path and a portion connected to the reaction chamber portion have hydrophobicity, the plurality of second flow paths can have hydrophobicity, and unnecessary inflow of the liquid from the first flow path to the second flow paths and the backflow of the liquid from the reaction chamber portions to the second flow paths can be reliably restricted.

According to the flow path device of claim 8, since at least a part of each of inner walls of the plurality of reaction chamber portions has hydrophilicity, the plurality of reaction chamber portions can have hydrophilicity. Therefore, since the reaction chamber portion is a place in which a desired reaction is performed on the liquid which is a sample, and the liquid is normally an aqueous medium, the liquid can flow into the reaction chamber portion and the desired reaction can be performed.

### Brief Description of the Drawings

Fig. 1 is a view illustrating a flow path device according to a first embodiment of the invention, Fig. 1(a) is a plan view, and Fig. 1(b) is a front view.
Fig. 2 is a view illustrating a state where a lid portion of the flow path device of Fig. 1 is removed, Fig. 2(a) is a plan view, and Fig. 2(b) is a cross-sectional view taken along line A-A in Fig. 2(a).
Fig. 3 is a front view illustrating a state where a pipette tip is inserted into the flow path device of Fig. 2 and a temperature regulating unit is attached to the flow path device.
Fig. 4 is a view illustrating an injection step of a test method according to the first embodiment, and is a plan view illustrating a state where a liquid is injected into each inlet portion side first flow path (partially not illustrated).
Fig. 5 is a view illustrating the injection step of the test method using the flow path device according to the first embodiment of the invention, and is a plan view illustrating a state after a positive pressure is applied to the liquid in each inlet portion side first flow path (partially not illustrated).
Fig. 6 is a view illustrating a flow path device according to a second embodiment of the invention, Fig. 6(a) is a plan view, and Fig. 6(b) is a cross-sectional view taken along line B-B in Fig. 6(a).

### Mode for Carrying Out the Invention

Hereinafter, embodiments of a flow path device and a test system according to the invention will be described in detail with reference to the accompanying drawings. First, [I] a basic concept of the embodiments will be described, and then [II] specific contents of the embodiments will be described, and finally, [III] modification examples of the embodiments will be described. Meanwhile, the invention is not limited by the embodiments.

### [I] Basic Concept of Embodiments

First, the basic concept of the embodiments will be described. The embodiments relate generally to the flow path device and the test system that contain a liquid containing a test object to be used for a biochemical test, a hematology test, a nucleic acid test, or an immunological test.

Here, the "biochemical test" means a test for measuring components in a sample such as blood using a colorimetric method, a turbidity measurement method, an enzyme method, an enzyme activity method, or the like. The items to be measured by the "biochemical test" are arbitrary items, and exemplary examples of the items include AST, ALT, and γ-GT in a liver function test, LDL cholesterol, HDL cholesterol, and triglyceride in a lipid test, blood glucose and HbAlc in a diabetes test, and the like. In addition, the "hematology test" means a test for quantitatively and qualitatively testing components in blood, mainly blood cell components and plasma components. The items to be measured by the "hematology test" are arbitrary items, and exemplary examples of the items include red blood cell count, hemoglobin content, white blood cell count, hematocrit, platelet count, and the like. In addition, the "nucleic acid test" is a test for detecting or quantifying a specific deoxyribonucleic acid (DNA) or ribonucleic acid (RNA) in a sample. As representative nucleic acid tests, there are known a DNA probe method, a southern hybridization method, a northern hybridization method, and the like in addition to a polymerase chain reaction method (PCR method), a TMA method, an NASBA method, an LAMP method, and the like which are nucleic acid amplification methods. In addition, the "immunological test" means a test for measuring or detecting components in a sample such as urine or blood using antigen-antibody reaction. Exemplary examples of the types of the "immunological test" to which the invention is applicable include a nefelometry method, an immunoturbidimetric method, an aggregation method, and the like. In addition, the items to be measured by the "immunological test" are arbitrary items, and exemplary examples of the items include pathogens or viruses of infectious diseases, antibodies, hormones, cancer markers, autoantibodies thereagainst, and the like.

In addition, exemplary examples of the types of "test objects to be used for a biochemical test, a hematology test, a nucleic acid test, or an immunological test" include serum, plasma, whole blood, blood cell components, urine, stool, sputum, spinal fluid, oral mucosa, pharyngeal mucosa, intestinal mucosa, vaginal mucosa, and biopsy samples (for example, fine needle aspiration (FNA) samples, intestinal samples, and liver samples). Alternatively, exemplary examples thereof include processed samples obtained by processing the above test objects using an acid, an alkali, a protein denaturant, a surfactant, an oxidizing agent, a reducing agent, an enzyme, dilution, filtration, extraction, heating, concentration, or the like, or a combination thereof. In addition, a "liquid containing a test object" may be the test object itself or may be a mixture of the test object and a solid, semi-solid, or liquid substance or the like other than the test object.

In addition, the "test system" is a system that performs a biochemical test, a hematology test, a nucleic acid test, or an immunological test on the liquid discharged from a pipette tip to be described later to the flow path device. Hereinafter, in the embodiments, a case will be described in which the test system is a system to be used for a nucleic acid test based on a real-time PCR method and the flow path device is a device that contains a processed sample subjected to a DNA extraction process and a real-time PCR reagent (including DNA polymerases, dNTP, primers, fluorescent labeled probes, and the like). Incidentally, the "real-time PCR method" is a type of PCR method for amplifying DNA or RNA, and is a method for monitoring and analyzing DNA or RNA while amplifying the DNA or RNA. In addition, exemplary examples of the types of the "real-time PCR method" include an intercalation method using a fluorescent substance or an electrochemical substance, a hybridization method using a fluorescent labeled probe, a turbidity detection method, an electrical detection method, and the like. In the embodiments, a description will be given based on the assumption that the hybridization method, particularly a TaqMan (registered trademark) method is used.

### [II] Specific Contents of Embodiments

Next, the specific contents of the embodiments will be described.

### [First Embodiment]

First, a first embodiment will be described. In the first embodiment, a first flow path and a second flow path are disposed at substantially the same height position.

### (Configuration)

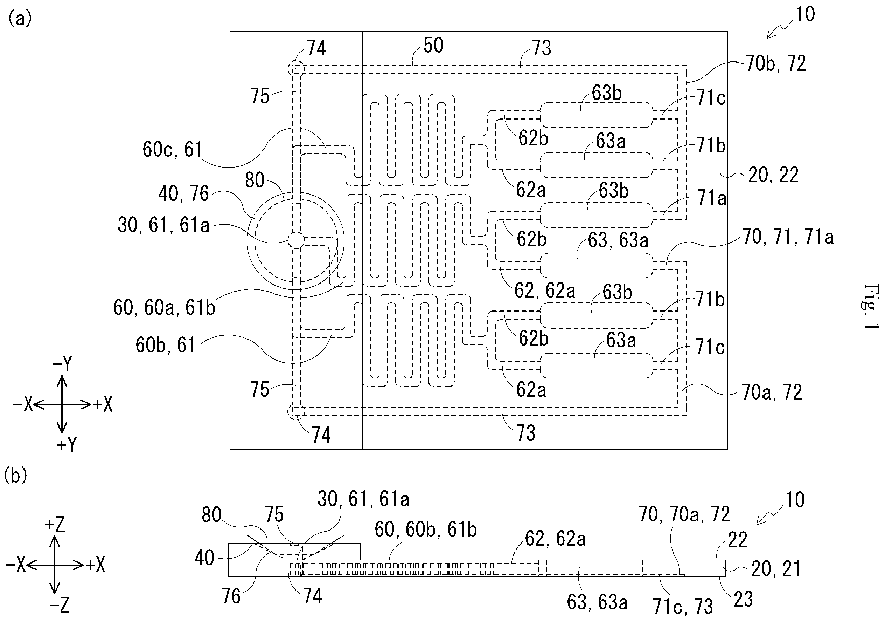

First, a configuration of a test system according to the first embodiment will be described. In the following description, an X direction in Fig. 1 is referred to as a right-left direction of a flow path device 10 to be described later (a -X direction is referred to as a left direction of the flow path device 10 and a +X direction is referred to as a right direction of the flow path device 10), a Y direction in Fig. 1 is referred to as a front-rear direction of the flow path device 10 (a +Y direction is referred to as a front direction of the flow path device 10 and a -Y direction is referred to as a rear direction of the flow path device 10), and a Z direction in Fig. 1 is referred to as an up-down direction of the flow path device 10 (a +Z direction is referred to as an up direction of the flow path device 10 and a -Z direction is referred to as a down direction of the flow path device 10).

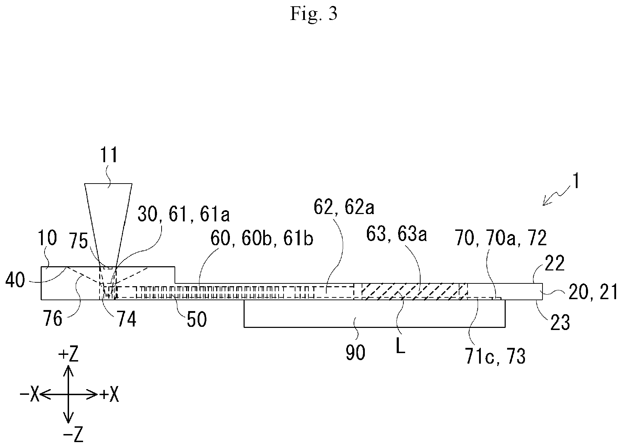

Generally, a test system 1 includes a discharge and suction unit (not illustrated), an attachment and detachment unit (not illustrated), a detection unit (not illustrated), and a control unit (not illustrated) in addition to the flow path device 10 and a temperature regulating unit 90 illustrated in Fig. 3.

In addition, a method for electrically connecting parts in the test system 1 is arbitrarily adopted. In the first embodiment, each of the discharge and suction unit, the attachment and detachment unit, the temperature regulating unit 90, and the detection unit is electrically connected to the control unit via wirings not illustrated. Accordingly, communication between each of the discharge and suction unit, the attachment and detachment unit, the temperature regulating unit 90, or the detection unit and the control unit or the supply of electric power can be directly or indirectly performed.

### (Configuration - Flow Path Device)

The flow path device 10 contains a liquid L containing a test object (for example, a processed sample containing DNA obtained by processing a biological sample) (hereinafter, referred to as the "liquid L"), and is provided on an installation surface not illustrated. Incidentally, details of a configuration of the flow path device 10 will be described later.

### (Configuration - Discharge and Suction Unit)

The discharge and suction unit is a discharge and suction device that discharges and suctions the liquid L or a gas (for example, air or the like) via a pipette tip 11 (for example, a pipette tip made of a resin material or a glass material). The discharge and suction unit is formed of, for example, a known dispensing device for testing (as one example, a dispensing device including a nozzle and a pump not illustrated) or the like, and is provided in the vicinity of the flow path device 10.

### (Configuration - Attachment and Detachment Unit)

The attachment and detachment unit is an attachment and detachment device used to attach or remove a lid portion 80 of the flow path device 10 to be described later to or from a main body portion 20 of the flow path device 10 to be described later. The attachment and detachment unit is formed of, for example, a known chuck mechanism for testing (as one example, a mobile chuck mechanism) or the like, and is provided in the vicinity of the flow path device 10.

### (Configuration - Detection Unit)

The detection unit is a detection device that detects a target component contained in the liquid L contained in the flow path device 10. The detection unit is formed of, for example, a known detection device (as one example, a spectrofluorometer) or the like, and is provided in the vicinity of the flow path device 10.

### (Configuration - Temperature Regulating Unit)

The temperature regulating unit 90 is a temperature regulating device that directly or indirectly heats or cools the flow path device 10. A plurality of the temperature regulating units 90 are provided in the vicinity of the flow path device 10, and specifically, as illustrated in Fig. 3, are provided at a position above, a position below, or positions above and below the flow path device 10 (in the illustrated example, provided only at a position therebelow). A specific configuration of the temperature regulating unit 90 is arbitrarily adopted. Either a heating device and a cooling device, for example, a Peltier element, an oil bath, air, or the like to be used for a normal real-time PCR reaction can be used, and in the illustrated example, is formed of a Peltier element capable of directly heating and cooling the flow path device 10.

### (Configuration - Control Unit)

The control unit is a unit that controls each part of the test system 1, and includes an operation unit; a communication unit; an output unit; a power supply unit; a controller; and a storage unit (all are not illustrated).

### (Configuration - Control Unit - Operation Unit, Communication Unit, Output Unit, and Power Supply Unit)

The communication unit is a communication device that enables communication between the discharge and suction unit, the attachment and detachment unit, the temperature regulating unit 90, and the detection unit. The output unit is an output device that outputs various information based on control of the control unit, and is formed of, for example, a known display device or audio output device. The power supply unit is a power supply device that supplies electric power, which is supplied from a commercial power supply or an electric cell (for example, a battery or the like), to each part of the control unit and supplies electric power to the discharge and suction unit, the attachment and detachment unit, the temperature regulating unit 90, and the detection unit.

### (Configuration - Control Unit - Controller)

The controller is a control device that controls each part of the control unit. Specifically, the controller is a computer including a CPU, various programs (including a basic control program such as an OS and an application program that is started on the OS to realize a specific function) to be interpreted and executed on the CPU, and an internal memory such as a RAM that stores the programs or various data.

### (Configuration - Control Unit - Storage Unit)

The storage unit is a storage device that stores programs and various data required for the operation of the control unit, and is formed of a known rewritable recording medium.
For example, a non-volatile recording medium such as a flash memory can be used.

### (Configuration - Details of Configuration of Flow Path Device)

Returning to Fig. 1, next, the details of the configuration of the flow path device 10 will be described. Meanwhile, unless otherwise specified, the flow path device 10 can be produced from any material in any shape by any method.

In the first embodiment, as illustrated in Fig. 1, the flow path device 10 includes the main body portion 20; an inlet portion 30; an outlet portion 40; a flow path 50; and the lid portion 80.

### (Configuration - Details of Configuration of Flow Path Device - Main Body Portion)

The main body portion 20 is a basic structure of the flow path device 10, and as illustrated in Figs. 1 to 3, includes a first main body portion 21; an upper second main body portion 22; and a lower second main body portion 23.

### (Configuration - Details of Configuration of Flow Path Device - Main Body Portion - First Main Body Portion)

The first main body portion 21 is a part of a basic structure of the main body portion 20, and includes the flow path 50. The first main body portion 21 is formed in a substantially plate shape (in the illustrated example, formed as a plate-shaped body of which the plan shape is a rectangular shape and the side shape is a substantially L shape), and as illustrated in Fig. 1, is provided approximately horizontally.

In addition, the size of the first main body portion 21 can be arbitrarily set as long as the first main body portion 21 can contain the flow path 50, and in the illustrated example, is set as follows. Namely, as illustrated in Fig. 1, the length in the right-left direction of the first main body portion 21 is set to be longer than the length in the right-left direction of the flow path 50, and the length in the front-rear direction of the first main body portion 21 is set to be longer than the length in the front-rear direction of the flow path 50. In the illustrated example, the length in the up-down direction of the first main body portion 21 is substantially the same as the length in the up-down direction of the flow path 50.

### (Configuration - Details of Configuration of Flow Path Device - Main Body Portion - Upper Second Main Body Portion and Lower Second Main Body Portion)

The upper second main body portion 22 and the lower second main body portion 23 are another part of the basic structure of the main body portion 20, and close open surfaces of the flow path 50. In the illustrated example, the upper second main body portion 22 and the lower second main body portion 23 are formed separately from the first main body portion 21, are formed in a thin layer shape (specifically, formed as a rectangular thin film-shaped body), and are attached to respective main side surfaces of the first main body portion 21 (side surfaces having the largest area among side surfaces of the first main body portion 21). Specifically, as illustrated in Fig. 1, the upper second main body portion 22 is attached to an upper surface of the first main body portion 21 by bonding, welding, or the like and the lower second main body portion 23 is attached to a lower surface of the first main body portion 21 by bonding, welding or the like.

In addition, the size of the upper second main body portion 22 and the lower second main body portion 23 can be arbitrarily set as long as the upper second main body portion 22 and the lower second main body portion 23 can close the open surfaces of the flow path 50, and in the illustrated example, is set as follows. Namely, as illustrated in Fig. 1, the length in the right-left direction of each of the upper second main body portion 22 and the lower second main body portion 23 is set to be substantially the same as the length in the right-left direction of the first main body portion 21. The length in the front-rear direction of each of the upper second main body portion 22 and the lower second main body portion 23 is set to be substantially the same as the length in the front-rear direction of the first main body portion 21. The length in the up-down direction of each of the upper second main body portion 22 and the lower second main body portion 23 is set to be shorter than the length in the up-down direction of the first main body portion 21. Incidentally, the "upper second main body portion 22" and the "lower second main body portion 23" described above correspond to a "second main body portion" in the claims.

### (Configuration - Details of Configuration of Flow Path Device - Main Body Portion - Oher Configurations)

In addition, the material of the flow path device 10 is not particularly limited; however, for example, when the flow path device 10 is used for real-time PCRs, in order to detect fluorescence, it is preferable that a translucent material (transparent material) is used. Exemplary examples of such a material include resin materials such as polypropylene, polystyrene, polyethylene, polycarbonate, an acrylic resin (polymethylmethacrylate (PMMA)), cyclic olefin resins (cycloolefin polymer (COP) and cyclic olefin copolymer (COC)), a silicone resin (PDMS), polyethylene terephthalate (PET), a photosensitive epoxy resin (SU-8), glass, and the like. Further, it is preferable that a material having low autofluorescence is used. Particularly, a cyclic olefin resin, a cyclic olefin copolymer, or quartz glass can be suitably used. Hereinafter, a description will be given based on the assumption that a cyclic olefin resin, polyophilene, or the like which is a hydrophobic material used as the material of the flow path device 10.

Since the main body portion 20 is configured in such a manner, even when a configuration of the flow path 50 is complicated, the main body portion 20 can be simply configured, and the ease of production of the flow path device 10 can be improved.

### (Configuration - Details of Configuration of Flow Path Device - Inlet Portion and Outlet Portion)

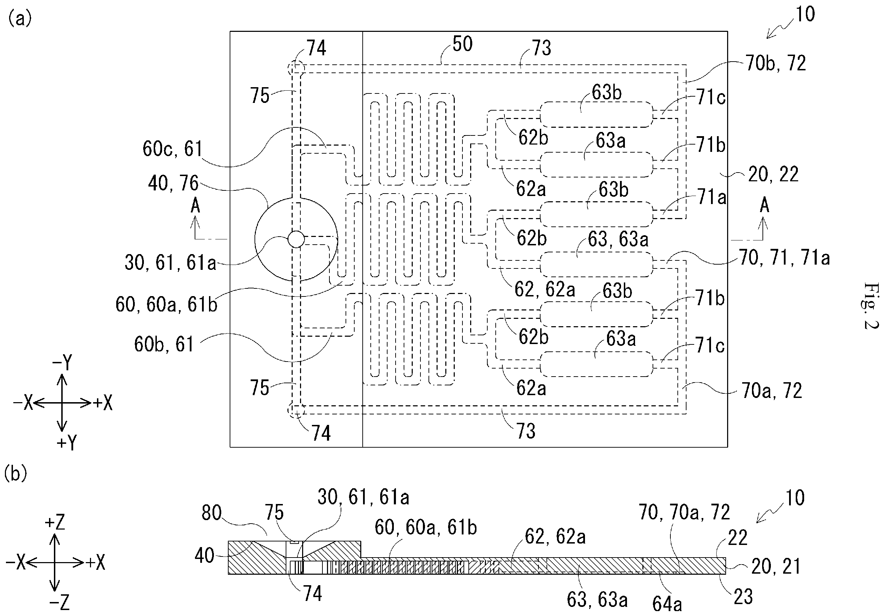

The inlet portion 30 is an opening which is such that the liquid L flows into the flow path 50 via the inlet portion 30, and the outlet portion 40 is such that air in the flow path 50 flows out to the outside via the outlet portion 40. In the example illustrated in Fig. 2, the inlet portion 30 and the outlet portion 40 each are formed in a circular shape and provided so as to be exposed to the outside of the main body portion 20. In the illustrated example, the inlet portion 30 is provided over the first main body portion 21 and the upper second main body portion 22 in a left portion of the main body portion 20, and is connected to the flow path 50 (specifically, an upstream end portion of the flow path 50). In addition, the outlet portion 40 is provided over the first main body portion 21 and the upper second main body portion 22 on a left side of the main body portion 20, and is connected to the flow path 50 (specifically, a downstream end portion of the flow path 50).

In addition, a method for installing the inlet portion 30 and the outlet portion 40 is arbitrarily adopted. In the example illustrated in Fig. 2, the inlet portion 30 and the outlet portion 40 are concentrically (specifically, concentrically when seen in a plan direction) disposed, and specifically, are disposed such that a center point of the inlet portion 30 is located directly below a center point of the outlet portion 40. In this case, the sizes of the diameters of the inlet portion 30 and the outlet portion 40 are arbitrarily set. In the example illustrated in Fig. 2, the diameter of the inlet portion 30 is set to be larger than the outer diameter of a tip portion of the pipette tip 11 through which the liquid L is injected, and is smaller than the maximum outer diameter of the pipette tip 11. Here, normally, the pipette tip 11 is configured such that the outer diameter of the tip portion at which a suction and discharge port is present is the smallest and the outer diameter gradually increases as the distance from the tip portion increases. For this reason, when the pipette tip 11 is inserted into the inlet portion 30, an outer peripheral portion of the pipette tip 11 comes into with contact with an inner wall of a first flow path side connection portion 61a to be described later without a tip of the pipette tip 11 coming into contact with a downstream end portion of the first flow path side connection portion 61a to be described later. Accordingly, when the pipette tip 11 is inserted into the inlet portion 30, an outer wall of the pipette tip 11 comes into contact with an inner wall of an inlet portion side flow path 60 to be described later, so that the inlet portion side flow path 60 to be described later can be sealed with the pipette tip 11. However, the invention is not limited thereto. For example, the diameter of the inlet portion 30 may be the diameter of the outlet portion 40 or greater.

### (Configuration - Details of Configuration of Flow Path Device - Flow Path)

Returning to Fig. 1, the flow path 50 contains the liquid L, which is discharged from the pipette tip 11, so as to be flowable. The flow path 50 is formed as a hollow portion (as one example, a quadrangular columnar hollow portion) and as illustrated in Fig. 1, provided inside the main body portion 20, and includes the inlet portion side flow path 60 and an outlet portion side flow path 70.

### (Configuration - Details of Configuration of Flow Path Device - Flow Path - Inlet portion Side Flow Path)

The inlet portion side flow path 60 is a flow path located on an inlet portion 30 side among portions of the flow path 50, and is a flow path that contains the liquid L. As illustrated in Figs. 1 and 2, the inlet portion side flow path 60 is provided inside the first main body portion 21, and includes a first inlet portion side flow path 60a located on a center side; a second inlet portion side flow path 60b located on a front side of the first inlet portion side flow path 60a; and a third inlet portion side flow path 60c located on a rear side of the first inlet portion side flow path 60a. Incidentally, hereinafter, since configurations of the first inlet portion side flow path 60a, the second inlet portion side flow path 60b, and the third inlet portion side flow path 60c are substantially the same, only the configuration of the first inlet portion side flow path 60a will be described below.

### (Configuration - Details of Configuration of Flow Path Device - Flow Path - Inlet Portion Side Flow Path - First Inlet Portion Side Flow Path)

As illustrated in Figs. 1 and 2, the first inlet portion side flow path 60a includes an inlet portion side first flow path 61; an inlet portion side second flow path 62; and reaction chamber portions 63a and 63b. Incidentally, when it is not particularly necessary to distinguish between the reaction chamber portions 63a and 63b, the reaction chamber portions 63a and 63b is collectively referred to simply as a "reaction chamber portion 63". In addition, the "inlet portion side first flow path 61" described above corresponds to a "first flow path" in the claims, and the "inlet portion side second flow path 62" described above corresponds to a "second flow path" in the claims.

### (Configuration - Details of Configuration of Flow Path Device - Flow Path - Inlet Portion Side Flow Path - First Inlet Portion Side Flow Path - Inlet Portion Side First Flow Path)

Returning to Fig. 1, the inlet portion side first flow path 61 is a part of a basic structure of the first inlet portion side flow path 60a, and primarily dispenses the liquid L. As illustrated in Fig. 1, the inlet portion side first flow path 61 is provided from the vicinity of an upper end portion of the first main body portion 21 to a lower end portion, is connected to the inlet portion 30, and includes the first flow path side connection portion 61a and a first flow path side main body portion 61b.

The first flow path side connection portion 61a of these portions is a portion that connects the inlet portion 30 and the first flow path side main body portion 61b, as illustrated in Fig. 1, projects from the inlet portion 30 toward an upstream end portion of the first flow path side main body portion 61b, and is disposed such that an axial direction of the first flow path side connection portion 61a is along the right-left direction. In addition, in the illustrated example, the first flow path side connection portion 61a is formed so as to serve also as the first flow path side connection portion 61a of the second inlet portion side flow path 60b and the third inlet portion side flow path 60c. However, the invention is not limited thereto, and the first flow path side connection portion 61a may be formed separately from the first flow path side connection portion 61a of the second inlet portion side flow path 60b or the third inlet portion side flow path 60c. In addition, the first flow path side main body portion 61b is a basic structure of the inlet portion side first flow path 61, as illustrated in Fig. 1, projects from the downstream end portion of the first flow path side connection portion 61a toward an upstream end portion of the inlet portion side second flow path 62, and is disposed such that an axial direction of the first flow path side main body portion 61b lies horizontally. Incidentally, details of a configuration of the inlet portion side first flow path 61 will be described later.

### (Configuration - Details of Configuration of Flow Path Device - Flow Path - Inlet Portion Side Flow Path - First Inlet Portion Side Flow Path - Inlet Portion Side Second Flow Path)

The inlet portion side second flow path 62 is another part of the basic structure of the first inlet portion side flow path 60a, and connects the inlet portion side first flow path 61 and the reaction chamber portions 63a and 63b. As illustrated in Fig. 1, the inlet portion side second flow path 62 is provided from the vicinity of the upper end portion of the first main body portion 21 to the lower end portion at a position on a right side of the first inlet portion side flow path 60a (namely, disposed at substantially the same height position as that of the first inlet portion side flow path 60a), and includes a second flow path side first connection portion 62a and a second flow path side second connection portion 62b.

The second flow path side first connection portion 62a of these portions is a portion connected to the inlet portion side first flow path 61 (namely, one of a plurality of the inlet portion side first flow paths 61) of the first inlet portion side flow path 60a and the reaction chamber portion 63a, as illustrated in Fig. 1, is formed such that the plan shape is a curved shape (for example, a substantially L shape), and is connected to a downstream end portion of the inlet portion side first flow path 61 and an upstream end portion of the reaction chamber portion 63a. The second flow path side second connection portion 62b is a portion connected to the inlet portion side first flow path 61 (namely, one of the plurality of inlet portion side first flow paths 61) of the first inlet portion side flow path 60a and the reaction chamber portion 63b, as illustrated in Fig. 1, is formed such that the plan shape is a curved shape (for example, a substantially inverted L shape), and is connected to the downstream end portion of the inlet portion side first flow path 61 and an upstream end portion of the reaction chamber portion 63b.

### (Configuration - Details of Configuration of Flow Path Device - Flow Path - Inlet Portion Side Flow Path - First Inlet Portion Side Flow Path - Reaction Chamber Portion)

The reaction chamber portions 63a and 63b are another part of the basic structure of the first inlet portion side flow path 60a, and are used for the reaction of the liquid L. Each of the reaction chamber portions 63a and 63b is formed such that the plan shape is linear along the right-left direction, and as illustrated in Fig. 1, is provided from the upper end portion of the first main body portion 21 to the lower end portion at a position on a right side of the inlet portion side second flow path 62 (namely, each formed as a through-hole).

### (Configuration - Details of Configuration of Flow Path Device - Flow Path - Outlet Portion Side Flow Path)

The outlet portion side flow path 70 is a flow path located on an outlet portion 40 side among the portions of the flow path 50, and is a flow path that allows air in the flow path 50 to flow out to the outside. As illustrated in Figs. 1 and 2, the outlet portion side flow path 70 is provided inside the first main body portion 21, and includes a first outlet portion side flow path 70a located on a front side, and a second outlet portion side flow path 70b located on a rear side of the first outlet portion side flow path 70a. Incidentally, in the first embodiment, since configurations of the first outlet portion side flow path 70a and the second outlet portion side flow path 70b are substantially the same, hereinafter, only the configuration of the first outlet portion side flow path 70a will be described.

### (Configuration - Details of Configuration of Flow Path Device - Flow Path - Outlet Portion Side Flow Path - First Outlet Portion Side Flow Path)

Returning to Fig. 1, the first outlet portion side flow path 70a includes, as illustrated in Fig. 1, outlet portion side first flow paths 71a, 71b, and 71c; an outlet portion side second flow path 72; an outlet portion side third flow path 73; an outlet portion side fourth flow path 74; an outlet portion side fifth flow path 75; and an outlet portion side fifth flow path 76. Incidentally, when it is not particularly necessary to distinguish between the outlet portion side first flow paths 71a, 71b, and 71c, the outlet portion side first flow paths 71a, 71b, and 71c are collectively referred to simply as an "outlet portion side first flow path 71".

The outlet portion side first flow paths 71a, 71b, and 71c are a part of a basic structure of the first outlet portion side flow path 70a, and are portions connected to the first inlet portion side flow path 60a (specifically, the reaction chamber portion 63a) and the first inlet portion side flow path 60b (specifically, the reaction chamber portions 63a and 63b). Each of the outlet portion side first flow paths 71a, 71b, and 71c is formed such that the plan shape is linear along the right-left direction, and as illustrated in Fig. 1, is provided in the lower end portion of the first main body portion 21 and in the vicinity thereof at a position on a right side of the first inlet portion side flow path 60a and the first inlet portion side flow path 60b. Specifically, the outlet portion side first flow path 71a is connected to a downstream end portion of the reaction chamber portion 63a of the first inlet portion side flow path 60a, the outlet portion side first flow path 71b is connected to a downstream end portion of the reaction chamber portion 63b of the first inlet portion side flow path 60b, and the outlet portion side first flow path 71c is connected to the downstream end portion of the reaction chamber portion 63a of the first inlet portion side flow path 60b.

The outlet portion side second flow path 72 is another part of the basic structure of the first outlet portion side flow path 70a, and is a portion connected to the outlet portion side first flow paths 71a, 71b, and 71c. The outlet portion side second flow path 72 is formed such that the plan shape is linear along the front-rear direction. As illustrated in Fig. 1, the outlet portion side second flow path 72 is provided in the lower end portion of the first main body portion 21 and in the vicinity thereof at a position on a right side of the outlet portion side second flow path 72, and is connected to a downstream end portion of each of the outlet portion side first flow paths 71a, 71b, and 71c.

The outlet portion side third flow path 73 is another part of the basic structure of the first outlet portion side flow path 70a, and is a portion connected to the outlet portion side second flow path 72. The outlet portion side third flow path 73 is formed such that the plan shape is linear along the right-left direction. As illustrated in Fig. 1, the outlet portion side third flow path 73 is provided in the lower end portion of the first main body portion 21 and in the vicinity thereof at a position on a front side of the outlet portion side second flow path 72, and is connected to a downstream end portion of the outlet portion side second flow path 72.

The outlet portion side fourth flow path 74 is another part of the basic structure of the first outlet portion side flow path 70a, and is a portion connected to the outlet portion side third flow path 73. The outlet portion side fourth flow path 74 is formed such that the side shape is linear along the up-down direction, as illustrated in Fig. 1(b), is provided from the lower end portion of the first main body portion 21 to the upper end portion at a position on a left side of the outlet portion side third flow path 73 (namely, formed as a through-hole), and is connected to a downstream end portion of the outlet portion side third flow path 73.

The outlet portion side fifth flow path 75 is another part of the basic structure of the first outlet portion side flow path 70a, and is a portion connected to the outlet portion side fourth flow path 74. The outlet portion side fifth flow path 75 is formed such that the plan shape is linear along the front-rear direction. As illustrated in Fig. 1, the outlet portion side fifth flow path 75 is provided in the upper end portion of the first main body portion 21 and in the vicinity thereof at a position on a rear side of the outlet portion side fourth flow path 74, and is connected to a downstream end portion of the outlet portion side fourth flow path 74.

The outlet portion side sixth flow path 76 is another part of the basic structure of the first outlet portion side flow path 70a, and is a portion connected to the outlet portion side fifth flow path 75 and the outlet portion 40. The outlet portion side sixth flow path 76 is formed such that the side shape is linear along the up-down direction. As illustrated in Fig. 1(b), the outlet portion side sixth flow path 76 is provided from the outlet portion 40 of the first main body portion 21 to the inlet portion 30 at a position on a rear side of the outlet portion side fifth flow path 75, and is connected to a downstream end portion of the outlet portion side fifth flow path 75 and the outlet portion 40. In addition, in the illustrated example, the outlet portion side sixth flow path 76 is formed so as to serve also as the outlet portion side sixth flow path 76 of the second outlet portion side flow path 70b. However, the invention is not limited thereto, and outlet portion side sixth flow path 76 may be formed separately from the outlet portion side sixth flow path 76 of the second outlet portion side flow path 70b. Incidentally, details of a configuration of the outlet portion side flow path 70 will be described later.

### (Configuration - Details of Configuration of Flow Path Device - Lid Portion)

Returning to Fig. 1, the lid portion 80 opens and closes the inlet portion 30 and the outlet portion 40. In the illustrated example, the inlet portion 30 and the outlet portion 40 can be collectively opened and closed with one lid portion 80. As illustrated in Fig. 1, the lid portion 80 is formed in a truncated cone shape and disposed so as to be inserted into the outlet portion side fifth flow path 75 via the outlet portion 40.

In addition, the shape and the size of the lid portion 80 are arbitrarily set, and in the illustrated example, are set as follows. Namely, as illustrated in Fig. 1, the side shape of the lid portion 80 is set to a substantially truncated cone shape in which the length of an upper end portion is larger than the length of a lower end portion. In addition, the plan shape of the lid portion 80 is set to a substantially circular shape. In addition, the diameter of the lid portion 80 is set to such a size that when the lid portion 80 is inserted into the outlet portion side fifth flow path 75, the entirety of the lid portion 80 can come into close contact with the outlet portion 40. For example, the minimum diameter of the lid portion 80 is set to be smaller than the diameter of the outlet portion 40, and the maximum diameter of the lid portion 80 is set to be larger than the diameter of the outlet portion 40. In addition, the length in the up-down direction of the lid portion 80 is set to be shorter than (or substantially the same as) the length in the up-down direction of the outlet portion side fifth flow path 75.

As a material forming the lid portion 80, a material having good heat resistance and chemical resistance and having proper flexibility (or rigidity) or elasticity which enables the lid portion 80 to seal the outlet portion 40 can be suitably used. Specifically, exemplary examples of the material include silicone rubber, butyl rubber, nitrile rubber, natural rubber, synthetic natural rubber, butadiene rubber, styrene butadiene rubber, ethylene propylene rubber, chloroprene rubber, acrylic rubber, chlorosulfonated polyethylene rubber, urethane rubber, fluororubber, and the like.

### (Details of Configuration of Inlet Portion Side Flow Path)

Next, details of a configuration of the inlet portion side flow path 60 will be described. In the illustrated example, characteristics of the inlet portion side flow path 60 (specifically, characteristics of the inlet portion side first flow path 61 and the inlet portion side second flow path 62) are as illustrated below.

### (Details of Configuration of Inlet Portion Side Flow Path - First Characteristics)

Initially, among first characteristics of the inlet portion side flow path 60, regarding characteristics of the inlet portion side first flow path 61 of each of the first inlet portion side flow path 60a, the second inlet portion side flow path 60b, and the third inlet portion side flow path 60c, each of the inlet portion side first flow paths 61 has hydrophilicity, and is configured such that when a positive pressure or negative pressure is applied to the liquid L in each of the inlet portion side first flow paths 61 by a predetermined method (specifically, when a positive pressure or negative pressure is applied to the liquid L such that the liquid L in each of the inlet portion side first flow paths 61 flows out to the corresponding the reaction chamber portion 63), the amounts of the liquid L flowing out from the inlet portion side first flow paths 61 toward the corresponding reaction chamber portions 63 are substantially the same. Here, in this specification, the "hydrophilicity" means a state where a solid surface easily gets wet with water, and specifically, means a state where the water contact angle is less than 90 degrees. Meanwhile, in this specification, the "hydrophobicity" means a state where the water contact angle exceeds 90 degrees. The "water contact angle" referred to here is measured by a method according to JIS R3257: 1999.

Specifically, at least a part of an inner wall of each of the inlet portion side first flow paths 61 has hydrophilicity over the entire length thereof (specifically, the entire length in the axial direction). More specifically, a first main body portion 21 side portion of the inner wall of each of the inlet portion side first flow paths 61 has hydrophilicity over the entire length thereof, and a lower second main body portion 23 side portion of the inner wall of each of the inlet portion side first flow paths 61 is formed of a hydrophilic film over the entire length thereof. However, the invention is not limited thereto. For example, the first main body portion 21 side portion may have hydrophilicity and the lower second main body portion 23 side portion may be formed of a hydrophobic film (film made of a hydrophobic material such as polypropylene), or the first main body portion 21 side portion may not have hydrophilicity and the lower second main body portion 23 side portion may be formed of a hydrophilic film. Here, "having hydrophilicity" can be achieved, for example, by performing a hydrophilic treatment on a solid surface of a desired portion. In addition, the "hydrophilic treatment" is a treatment for increasing the wettability of the liquid L with respect to the solid surface, and exemplary examples of the hydrophilic treatment include an oxygen plasma treatment, a VUV light treatment, and the like. Accordingly, since each of the inlet portion side first flow paths 61 can have hydrophilicity, when the liquid L is injected into the inlet portion side flow path 60 via the inlet portion 30, the liquid L can evenly (specifically, at an equal flow rate) flow into the inlet portion side first flow paths 61 due to capillary force acting on each of the inlet portion side first flow paths 61. In addition, the plurality of inlet portion side first flow paths 61 can reliably have hydrophilicity, and the function of the plurality of inlet portion side first flow paths 61 can be reliably exhibited.

In addition, the shape of each of the inlet portion side first flow paths 61 is set as follows. Namely, as illustrated in Fig. 1, the shape of the first flow path side connection portion 61a of each of the inlet portion side first flow paths 61 is set to a linear shape. However, the invention is not limited thereto, and the shape of the first flow path side connection portion 61a may be set to a curved shape. In addition, the shape of the first flow path side main body portion 61b of each of the inlet portion side first flow paths 61 is set to a curved shape. Specifically, in order to make the inlet portion side first flow path 61 compact and increase the containing capacity, as illustrated in Fig. 1, the shape of a downstream portion of the first flow path side main body portion 61b is set to a meandering shape. However, the invention is not limited thereto. For example, the shape of the downstream portion may be set to a curved shape other than a meandering shape, or may be set to a linear shape.

In addition, the size of each of the inlet portion side first flow paths 61 is set as follows such that the containing capacity of each of the inlet portion side first flow paths 61 is substantially the same as the containing capacity of the corresponding reaction chamber portion 63. Namely, the hydraulic equivalent diameter (when a flow path having an arbitrary cross-sectional shape is converted into an equivalent circular tube, the diameter of the equivalent circular tube) of each of the inlet portion side first flow paths 61 is set such that the hydraulic equivalent diameters of the inlet portion side first flow paths 61 are the same. More specifically, the hydraulic equivalent diameter of each of the first flow path side connection portion 61a and the first flow path side main body portion 61b of each of the inlet portion side first flow paths 61 is set to be substantially the same as the diameter of the inlet portion 30 (however, the invention is not limited thereto, and the hydraulic equivalent diameter may be set to be smaller than or larger than the diameter of the inlet portion 30). In addition, the length (length in the axial direction) of each of the inlet portion side first flow paths 61 is set such that the lengths of the inlet portion side first flow paths 61 are the same. More specifically, the length of the first flow path side connection portion 61a of each of the inlet portion side first flow paths 61 is set to a length shorter than the length in the up-down direction of the first main body portion 21. In addition, the length of the first flow path side main body portion 61b of each of the inlet portion side first flow paths 61 is set to approximately half the length in the right-left direction of the first main body portion 21 (however, the invention is not limited thereto, and the length of the first flow path side main body portion 61b may be set to be longer or shorter than half the length in the right-left direction of the first main body portion 21). With such setting, the containing capacity of each of the inlet portion side first flow paths 61 can be set to be substantially the same as the containing capacity of the corresponding reaction chamber portion 63. In addition, the amounts of the liquid L flowing out from the plurality of inlet portion side first flow paths 61 can be made substantially the same, and when positive pressures or negative pressures are applied to the liquid L in the plurality of respective inlet portion side first flow paths 61, the magnitudes of the applied positive pressures or negative pressures can be made substantially the same.

In addition, among the first characteristics of the inlet portion side flow path 60, regarding characteristics of the inlet portion side second flow path 62 of each of the first inlet portion side flow path 60a, the second inlet portion side flow path 60b, and the third inlet portion side flow path 60c, a configuration is adopted in which when the magnitude of the positive pressure or negative pressure applied to the liquid L in each of the inlet portion side first flow paths 61 is less than a threshold value, the outflow of the liquid L from the inlet portion side first flow path 61, which is connected to each of the inlet portion side second flow paths 62, to the corresponding reaction chamber portion 63 is restricted, and when the magnitude of the applied positive pressure or negative pressure is the threshold value or greater, the outflow of the liquid L is allowed.

Specifically, in an inner wall of each of the inlet portion side second flow paths 62, at least a portion connected to the inlet portion side first flow path 61 and a portion connected to the reaction chamber portion 63 have hydrophobicity. More specifically, a first main body portion 21 side portion of the inner wall of each of the inlet portion side second flow paths 62 is made of a hydrophobic material (for example, a cyclic olefin resin or the like) over the entire length thereof, and a lower second main body portion 23 side portion of the inner wall of each of the inlet portion side second flow paths 62 is formed of a hydrophobic film over the entire length thereof. However, the invention is not limited thereto. For example, in the inner wall of each of the inlet portion side second flow paths 62, only the portion connected to the inlet portion side first flow path 61 and the portion connected to the reaction chamber portion 63 may have hydrophobicity. Accordingly, a plurality of the inlet portion side second flow paths 62 can have hydrophobicity, and unnecessary inflow of the liquid L from the inlet portion side first flow paths 61 to the inlet portion side second flow paths 62 and the backflow of the liquid L from the reaction chamber portions 63 to the inlet portion side second flow paths 62 can be reliably restricted.

In addition, the size of each of the inlet portion side second flow paths 62 is set as follows. Namely, the hydraulic equivalent diameter of each of the inlet portion side second flow paths 62 is set such that the hydraulic equivalent diameters of the inlet portion side second flow paths 62 are the same, and more specifically, are set to be substantially the same as the hydraulic equivalent diameter of the inlet portion side first flow path 61 (however, the invention is not limited thereto, and the hydraulic equivalent diameter of each of the inlet portion side second flow paths 62 may be set to be smaller than or larger than the hydraulic equivalent diameter of the inlet portion side first flow path 61). In addition, the length (length in the axial direction) of each of the inlet portion side second flow paths 62 is set that the lengths (lengths in the axial direction) of the inlet portion side second flow paths 62 are the same, and more specifically, is set to a length shorter than the length of the inlet portion side first flow path 61. With such setting, when positive pressures or negative pressures are applied to the liquid L in the respective inlet portion side second flow paths 62, the magnitudes of the applied positive pressures or negative pressures can be made substantially the same.

Due to the first characteristics described above, when the liquid L is dispensed to the plurality of inlet portion side first flow paths 61 via the inlet portion 30, and a positive pressure or negative pressure is applied to the dispensed liquid L, the liquid L can flow out to a plurality of the reaction chamber portions 63, and the labor of work for dispensing the liquid L can be further reduced as compared to the technique of the related art (technique in which the supply port and the discharge port are provided in each of the plurality of flow paths). Particularly, since the plurality of inlet portion side first flow paths 61 can be configured such that the amounts of the liquid L flowing out from the plurality of inlet portion side first flow paths 61 are substantially the same, the liquid L can evenly flow out to the plurality of reaction chamber portions 63, and quantitative dispensing to the plurality of reaction chamber portions 63 can be realized. In addition, the plurality of inlet portion side second flow paths 62 can be configured such that when the magnitude of the positive pressure or negative pressure applied to the liquid L in the plurality of inlet portion side first flow paths 61 is less than the threshold value, the outflow of the liquid L is restricted, and when the magnitude of the applied positive pressure or negative pressure is the threshold value or greater, the outflow of the liquid L is allowed. Regardless of the number of the inlet portion side first flow paths 61 that are installed, quantitative dispensing to the plurality of reaction chamber portions 63 can be reliably performed. For the above reasons, the ease of use of the flow path device 10 can be improved.

### (Details of Configuration of Inlet Portion Side Flow Path - Second Characteristics)

Next, regarding second characteristics of the inlet portion side flow path 60 (specifically, characteristics of the reaction chamber portion 63), first, the number of the reaction chamber portions 63 connected to each of the inlet portion side second flow paths 62 is set to a power of 2. Specifically, as illustrated in Fig. 1, the number of the reaction chamber portions 63 is set to 2. With such setting, the liquid L can evenly flow into the reaction chamber portions 63 connected to each of the plurality of inlet portion side second flow paths 62, and quantitative dispensing to the plurality of reaction chamber portions 63 is easily realized. However, the invention is not limited thereto. For example, the number of the reaction chamber portions 63 may be set to other powers of 2, and as one example, may be set to 2 or more (4, 8, or the like) or may be set to 1.

In addition, at least a part of an inner wall of each of the reaction chamber portions 63 of each of the first inlet portion side flow path 60a, the second inlet portion side flow path 60b, and the third inlet portion side flow path 60c has hydrophilicity over the entire length thereof. Specifically, an upper second main body portion 22 side portion of the inner wall of each of the reaction chamber portions 63 is made of a hydrophobic material (for example, a cyclic olefin resin or the like) over the entire length thereof, and the upper second main body portion 22 side portion and a lower second main body portion 23 side portion of the inner wall of each of the reaction chamber portions 63 is formed of a hydrophilic film over the entire length thereof. However, the invention is not limited thereto. For example, a hydrophilic treatment may be applied to a first main body portion 21 side portion of the inner wall of each of the reaction chamber portions 63, and the upper second main body portion 22 side portion and the lower second main body portion 23 side portion of the inner wall of each of the reaction chamber portions 63 may be formed of a hydrophilic film. Accordingly, the reaction chamber portions 63 can have hydrophilicity. Therefore, since the reaction chamber portion 63 is a place in which a desired reaction is performed on the liquid L which is a sample, and the liquid L is normally an aqueous medium, the liquid L can flow into the reaction chamber portion 63 and the desired reaction can be performed.

In this case, the size of the reaction chamber portion 63 of each of the first inlet portion side flow path 60a, the second inlet portion side flow path 60b, and the third inlet portion side flow path 60c is set as follows in the illustrated example. Namely, the hydraulic equivalent diameter of each of the reaction chamber portions 63 is set such that the hydraulic equivalent diameters of the inlet portion side second flow paths 62 are the same, and specifically, is set to be larger than the hydraulic equivalent diameter of the inlet portion side second flow path 62 (however, the invention is not limited thereto, and the hydraulic equivalent diameter of each of the reaction chamber portions 63 may be set to the hydraulic equivalent diameter of the inlet portion side second flow path 62 or less). In addition, the length (length in the axial direction) of each of the reaction chamber portions 63 is set that the lengths (lengths in the axial direction) of the reaction chamber portions 63 are the same, and specifically, is set to a length shorter than the length of the inlet portion side first flow path 61.

Due to the second characteristics described above, the ease of use and the ease of production of the flow path device 10 can be improved.

### (Details of Configuration of Outlet Portion Side Flow Path)

Next, the details of the configuration of the outlet portion side flow path 70 will be described. Regarding characteristics of the outlet portion side flow path 70, in the first embodiment, in an inner wall of each of the first outlet portion side flow path 70a and the second outlet portion side flow path 70b, at least a portion connected to the inlet portion side flow path 60 has hydrophobicity. Specifically, a first main body portion 21 side portion of the inner wall of each of the first outlet portion side flow path 70a and the second outlet portion side flow path 70b is made of a hydrophobic material (for example, a cyclic olefin resin or the like) over the entire length thereof, and a lower second main body portion 23 side portion of the inner wall is formed of a hydrophobic film over the entire length thereof. However, the invention is not limited thereto. For example, only a portion of the inner wall connected to the inlet portion side flow path 60 may be made of a hydrophobic material. Accordingly, the outlet portion side flow path 70 can have hydrophobicity, and air in the inlet portion side flow path 60 can flow out to the outside while the inflow of the liquid L to the outlet portion side flow path 70 is suppressed.

In this case, the size of each of the first outlet portion side flow path 70a and the second outlet portion side flow path 70b can be arbitrarily set as long as air in the first inlet portion side flow path 60a and the first inlet portion side flow path 60b can flow out to the outside, and in the first embodiment, is set as follows. Namely, the hydraulic equivalent diameter of the outlet portion side first flow path 71, the outlet portion side second flow path 72, and the outlet portion side third flow path 73 of each of the first outlet portion side flow path 70a and the second outlet portion side flow path 70b is set to be smaller than the hydraulic equivalent diameter of the reaction chamber portion 63. In addition, the hydraulic equivalent diameter of the outlet portion side fourth flow path 74 of each of the first outlet portion side flow path 70a and the second outlet portion side flow path 70b is set to be larger than the hydraulic equivalent diameter of the outlet portion side third flow path 73 (however, the invention is not limited thereto, and the hydraulic equivalent diameter of the outlet portion side fourth flow path 74 may be set to be the hydraulic equivalent diameter of the outlet portion side third flow path 73 or less). In addition, the hydraulic equivalent diameter of the outlet portion side fifth flow path 75 of each of the first outlet portion side flow path 70a and the second outlet portion side flow path 70b is set to be smaller than the hydraulic equivalent diameter of the outlet portion side fourth flow path 74 (however, the invention is not limited thereto, and the hydraulic equivalent diameter of the outlet portion side fifth flow path 75 may be set to the hydraulic equivalent diameter of the outlet portion side fourth flow path 74 or less). In addition, the length (length in the axial direction) of the outlet portion side sixth flow path 76 of each of the first outlet portion side flow path 70a and the second outlet portion side flow path 70b is set to be larger than the hydraulic equivalent diameter of the outlet portion side fifth flow path 75. More specifically, as illustrated in Fig. 1(b), the hydraulic equivalent diameter of the outlet portion side sixth flow path 76 is set to increase toward the outlet portion 40. In addition, the length (length in the axial direction) of each of the first outlet portion side flow path 70a and the second outlet portion side flow path 70b is set to a length longer than the length in the right-left direction of the main body portion 20 (however, the invention is not limited thereto, and the length may be set to the length in the right-left direction of the main body portion 20 or less).

Due to the fourth characteristics described above, the ease of use and the ease of production of the flow path device 10 is easily further improved.

### (Test Method)

Subsequently, a specimen test method performed using the test system 1 of the invention will be described. The test method according to the first embodiment includes an injection step; an amplification step; and a detection step.

In addition, the assumptions of the test method are as follows. Namely, it is assumed that the flow path device 10 is assembled in advance. In addition, it is assumed that real-time PCR reagents (for example, DNA polymerases, dNTP, primers, TaqMan (registered trademark), probes, and the like) corresponding to different analysis items (including one or a plurality of different primer sets) are contained in the respective reaction chamber portions 63 of each of the first inlet portion side flow path 60a, the second inlet portion side flow path 60b, and the third inlet portion side flow path 60c in the flow path device 10 in advance. In addition, as illustrated in Fig. 3, it is assumed that the flow path device 10 is installed in a state where the flow path device 10 is in contact with the temperature regulating unit 90. In addition, it is assumed that control of each part of the test system 1 is performed by a control device. Accordingly, a plurality of types of real-time PCRs can be executed on the same sample at the same time by introducing substantially the same amount of the liquid L, which is the same sample, into the plurality of reaction chamber portions 63. More suitably, since different multiplex real-time PCR reagents exist in the respective reaction chamber portions 63, a larger number of types of real-time PCRs can be performed. Incidentally, the reagents existing in the reaction chamber portions 63 are not limited to the real-time PCR reagents, and may be reagents for biochemical tests, hematology tests, immunological tests, or other nucleic acid tests.

### (Test Method - Injection Step)

Initially, the injection step will be described. The injection step is a step of injecting the liquid L into the flow path device 10.

Specifically, first, after the liquid L is suctioned into the pipette tip 11 attached to the nozzle of the discharge and suction unit and then the nozzle and the pipette tip 11 are moved to above the inlet portion 30 of the flow path device 10, the pipette tip 11 is moved downward and inserted into the inlet portion side first flow path 61 of the inlet portion side flow path 60 via the inlet portion 30.

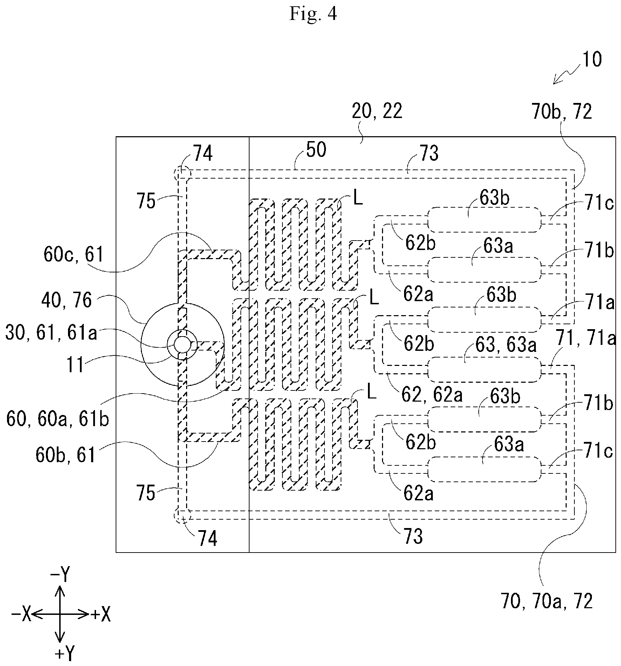

Next, as illustrated in Fig. 4, a predetermined amount of the liquid L is discharged from the nozzle of the discharge and suction unit via the pipette tip 11, so that the liquid L is injected into the inlet portion side first flow path 61 of each of the first inlet portion side flow path 60a, the second inlet portion side flow path 60b, and the third inlet portion side flow path 60c via the inlet portion 30. The predetermined amount is arbitrarily set, and for example, may be set to such an amount that the reaction chamber portions 63 of each of the first inlet portion side flow path 60a, the second inlet portion side flow path 60b, and the third inlet portion side flow path 60c can be substantially full. In addition, in this case, since the magnitude of the positive pressure applied to the liquid L in each of the inlet portion side first flow paths 61 along with the injection of the liquid L is less than the threshold value, the outflow of the liquid L from each of the inlet portion side first flow paths 61 to the corresponding the reaction chamber portion 63 is restricted by from each of the inlet portion side first flow paths 61 to the corresponding inlet portion side second flow path 62.

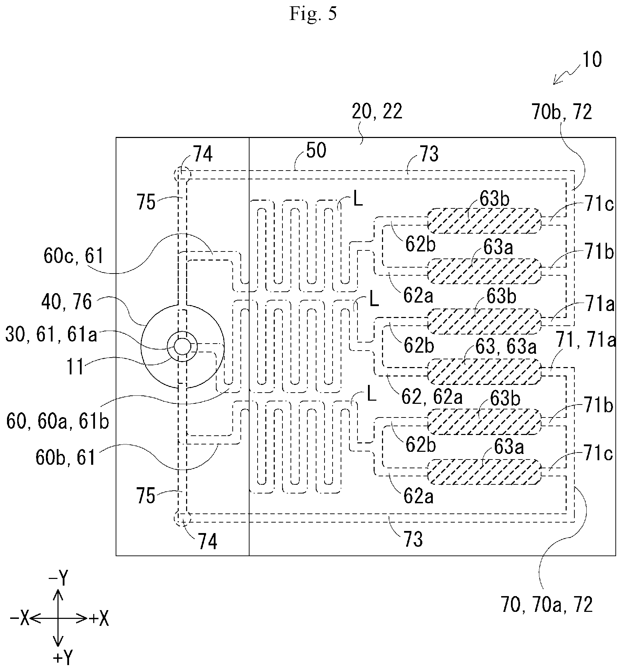

Next, as illustrated in Fig. 5, a positive pressure is applied to the liquid L in each of the inlet portion side first flow paths 61 by causing air to be discharged from the nozzle of the discharge and suction unit via the pipette tip 11. Then, the magnitude of the positive pressure applied to the liquid L in each of the inlet portion side first flow paths 61 is the threshold value or greater, so that the liquid L flows out from each of the inlet portion side first flow paths 61 to the corresponding reaction chamber portion 63, and the application of the positive pressure continues to be performed until all the liquid L in each of the inlet portion side first flow paths 61 flows out.

Thereafter, the pipette tip 11 is removed from the flow path device 10 by moving the nozzle of the discharge and suction unit upward and then in a horizontal direction.

The labor of work for dispensing the liquid L can be further reduced by such an injection step as compared to the technique of the related art (technique in which the supply port and the discharge port are provided in each of the plurality of flow paths). In addition, since the inlet portion 30 is a dispensing opening through which the liquid L is dispensed to the plurality of inlet portion side first flow paths 61 and is an application opening through which a positive pressure is applied to the liquid L in the plurality of inlet portion side first flow paths 61, the dispensing of the liquid L or the application of a positive pressure can be performed via the inlet portion 30, and the labor of work for dispensing the liquid L and work for applying a positive pressure can be further reduced as compared to when the dispensing opening and the application opening are individually provided.

### (Test Method - Amplification Step and Detection Step)

Next, the amplification step and the detection step in the real-time PCR method will be described. The amplification step is a step of amplifying a target sequence of DNA (or RNA) contained in the liquid L contained in each of the reaction chamber portions 63 after the injection step. The detection step is a step of detecting the target sequence of the amplified DNA after or during the amplification step. As described above, in the first embodiment, in order to perform a nucleic acid amplification test based on the real-time PCR method (an intercalation method or a fluorescent labeled probe hybridization method), the amplification step and the detection step are executed in parallel.

The lid portion 80 of the flow path device 10 is inserted into the outlet portion side fifth flow path 75 via the outlet portion 40 using the attachment and detachment unit. Accordingly, in the illustrated example, the outlet portion 40 and the inlet portion 30 are collectively closed with the lid portion 80. Next, the temperature regulating unit 90 and the detection unit are operated to detect fluorescence generated from the temperature regulation of the liquid L (particularly, the liquid L in the reaction chamber portions 63) contained in the flow path device 10 and the amplification of DNA. The nucleic acid amplification is executed by performing temperature regulation in a cycle of denaturation temperature (approximately 95°C) - annealing temperature (approximately 50 to 75°C) - elongation temperature (approximately 60 to 75°C). The detailed denaturation temperature, annealing temperature, and extension temperature, and the retention time at each temperature are adjusted and set in advance according to a template DNA, a primer sequence, and a target sequence. The above temperature cycle is executed normally approximately 25 to 50 times.

### (Test Method - Disposal Step)

Next, a disposal step will be described. The disposal step is a step of disposing the flow path device 10 after the amplification step and the detection step.

Specifically, after the detection step, the liquid L in the flow path device 10 can be provided for a post analysis, but the entirety of the flow path device 10 is disposed without opening the lid portion 80. Since disposal can be performed without opening the lid portion 80, a large amount of nucleic acid is prevented from diffusing into the environment in aerosols or the like after the amplification reaction. Therefore, the risk of contamination with other unreacted reagent solutions can be reduced. Particularly, it can be said that the effect of reducing the risk of contamination is large in the nucleic acid amplification test in which the target sequence of DNAis amplified several million times.

According to the above-described test method, the nucleic acid amplification test can be automatically performed by the test system 1, and the nucleic acid amplification test can be simply performed.

### (Effects of First Embodiment)

According to the first embodiment, the flow path 50 includes the plurality of inlet portion side first flow paths 61 connected to the inlet portion 30 and having hydrophilicity; the plurality of inlet portion side second flow paths 62 each of which is connected to one of the plurality of inlet portion side first flow paths 61; and the plurality of reaction chamber portions 63 each of which is used for the reaction of the liquid L and is connected to one of the plurality of inlet portion side second flow paths 62. The plurality of inlet portion side first flow paths 61 are configured such that when a positive pressure or negative pressure is applied to the liquid L in the plurality of inlet portion side first flow paths 61 by the predetermined method, the amounts of the liquid L flowing out from the plurality of inlet portion side first flow paths 61 toward the corresponding reaction chamber portions 63 are substantially the same. The plurality of inlet portion side second flow paths 62 are configured such that when the magnitude of the positive pressure or negative pressure applied to the liquid L in the plurality of inlet portion side first flow paths 61 is less than the threshold value, the outflow of the liquid L from the inlet portion side first flow path 61, which is connected to each of the plurality of inlet portion side second flow paths 62, to the corresponding reaction chamber portion 63 is restricted, and when the magnitude of the applied positive pressure or negative pressure is the threshold value or greater, the outflow of the liquid L is allowed. Therefore, when the liquid L is dispensed to the plurality of inlet portion side first flow paths 61 via the inlet portion 30, and a positive pressure or negative pressure is applied to the dispensed liquid L, the liquid L can flow out to the plurality of reaction chamber portions 63, and the labor of work for dispensing the liquid L can be further reduced as compared to the technique of the related art (technique in which the supply port and the discharge port are provided in each of the plurality of flow paths). Particularly, since the plurality of inlet portion side first flow paths 61 can be configured such that the amounts of the liquid L flowing out from the plurality of inlet portion side first flow paths 61 are substantially the same, the liquid L can evenly flow out to the plurality of reaction chamber portions 63, and quantitative dispensing to the plurality of reaction chamber portions 63 can be realized. In addition, the plurality of inlet portion side second flow paths 62 can be configured such that when the magnitude of the positive pressure or negative pressure applied to the liquid L in the plurality of inlet portion side first flow paths 61 is less than the threshold value, the outflow of the liquid L is restricted, and when the magnitude of the applied positive pressure or negative pressure is the threshold value or greater, the outflow of the liquid L is allowed. Regardless of the number of the inlet portion side first flow paths 61 that are installed, quantitative dispensing to the plurality of reaction chamber portions 63 can be reliably performed. For the above reasons, the ease of use of the flow path device 10 can be improved.

In addition, since the inlet portion 30 is a dispensing opening through which the liquid L is dispensed to the plurality of inlet portion side first flow paths 61 and is an application opening through which a positive pressure is applied to the liquid L in the plurality of inlet portion side first flow paths 61, the dispensing of the liquid L or the application of a positive pressure can be performed via the inlet portion 30, and the labor of work for dispensing the liquid L and work for applying a positive pressure can be further reduced as compared to when the dispensing opening and the application opening are individually provided.

In addition, since the hydraulic equivalent diameters of the plurality of inlet portion side first flow paths 61 are the same, and the lengths of the plurality of inlet portion side first flow paths 61 are the same, the amounts of the liquid L flowing out from the plurality of inlet portion side first flow paths 61 can be made substantially the same, and when positive pressures or negative pressures are applied to the liquid L in the plurality of respective inlet portion side first flow paths 61, the magnitudes of the applied positive pressures or negative pressures can be made substantially the same, so that quantitative dispensing to the plurality of reaction chamber portions 63 is easily realized.

In addition, since the plurality of inlet portion side first flow paths 61 are formed in a meandering shape, it is possible to make the inlet portion side first flow paths 61 compact and increase the containing capacity, and the ease of use of the flow path device 10 is easily improved.

In addition, since at least a part of each of the inner walls of the plurality of inlet portion side first flow paths 61 has hydrophilicity over the entire length thereof, the plurality of inlet portion side first flow paths 61 can reliably have hydrophilicity, and the function of the plurality of inlet portion side first flow paths 61 can be reliably exhibited.

In addition, since the number of the reaction chamber portions 63 connected to each of the plurality of inlet portion side second flow paths 62 is set to a power of 2, the liquid L can evenly flow into the reaction chamber portions 63 connected to each of the plurality of inlet portion side second flow paths 62, and quantitative dispensing to the plurality of reaction chamber portions 63 is easily realized.

In addition, since in each of the inner walls of the plurality of inlet portion side second flow paths 62, at least the portion connected to the inlet portion side first flow path 61 and the portion connected to the reaction chamber portion 63 have hydrophobicity, the plurality of inlet portion side second flow paths 62 can have hydrophobicity, and unnecessary inflow of the liquid L from the first flow path to the inlet portion side second flow paths 62 and the backflow of the liquid L from the reaction chamber portions 63 to the inlet portion side second flow paths 62 can be reliably restricted.

In addition, since at least a part of each of the inner walls of the plurality of reaction chamber portions 63 has hydrophilicity, the plurality of reaction chamber portions 63 can have hydrophilicity. Therefore, since the reaction chamber portion 63 is a place in which a desired reaction is performed on the liquid L which is a sample, and the liquid L is normally an aqueous medium, the liquid L can flow into the reaction chamber portion 63 and the desired reaction can be performed.

### [Second Embodiment]

Next, a second embodiment will be described. In the second embodiment, the first flow path and the second flow path are disposed at different height positions. Meanwhile, configurations of the second embodiment are substantially the same as the configurations of the first embodiment unless otherwise specified. The same reference signs and/or names as those used in the first embodiment are assigned to substantially the same configurations as the configurations of the first embodiment, and description thereof will be omitted.

### (Configuration)

Initially, a configuration of a test system according to the second embodiment will be described. The test system 1 according to the second embodiment is configured in substantially the same manner as the test system 1 according to the first embodiment. Meanwhile, the configuration of the flow path device 10 is devised as illustrated below.

### (Configuration - Details of Configuration of Flow Path Device)

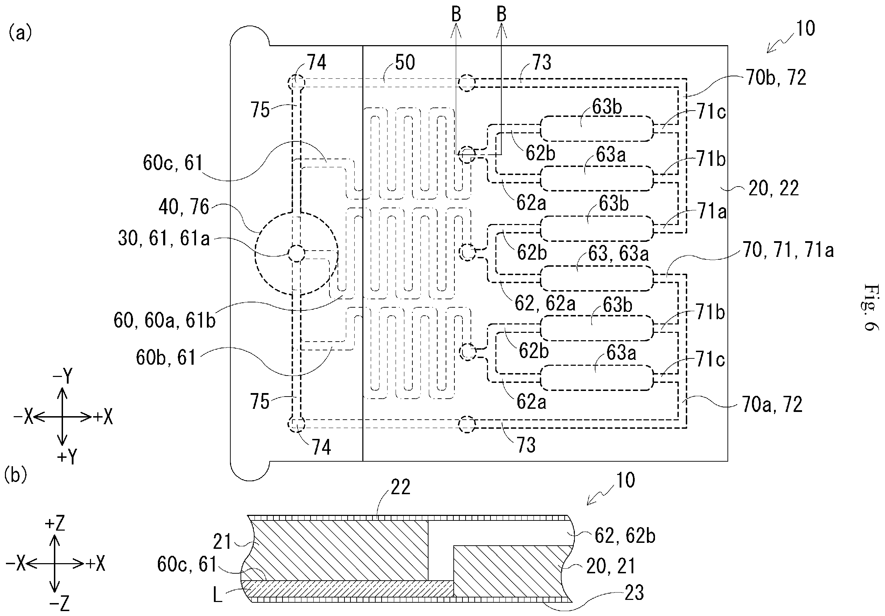

Next, the details of the configuration of the flow path device 10 will be described. In the second embodiment, as illustrated in Fig. 6, the main body portion 20 of the flow path device 10 is produced by separately producing the first main body portion 21, and the upper second main body portion 22 and the lower second main body portion 23 which have a film shape, and integrating both the upper second main body portion 22 and the lower second main body portion 23 with the first main body portion 21 by bonding, welding, or the like.

Here, among side surfaces of the upper second main body portion 22, a side surface to be bonded or welded to the first main body portion 21 is structured to have hydrophobicity as a whole. The hydrophobicity of this side surface is achieved by using a hydrophobic material or applying a hydrophobic treatment. Meanwhile, among side surfaces of the second main body portion 23, a side surface to be bonded or welded to the first main body portion 21 is structured to have hydrophilicity as a whole. The hydrophilicity of this side surface is achieved by applying a hydrophilic treatment or using a hydrophilic material.

In addition, the flow path 50 is formed by providing predetermined recesses and through-holes in the first main body portion 21 before integrating the second main body portions 22 and 23. In the flow path 50, a first inlet portion flow path 61 is formed as a groove-shaped recess in the lower surface of the first main body portion 21. In addition, since a second inlet portion flow path 62 is formed as a groove-shaped recess in the upper surface of the first main body portion 21, the first inlet portion flow path 61 and the second inlet portion flow path 62 can be disposed at different height positions. A connection portion between the first inlet portion flow path 61 and the second inlet portion flow path 62 is formed as a through-hole penetrating through the first main body portion 21 from the upper surface to the lower surface thereof. In addition, the reaction chamber portion 63 is formed as a through-hole, and the outlet portion side first flow path 71 is formed as a groove-shaped recess in the upper surface of the first main body portion 21. These flow paths can be formed by closing the grooves and the through-holes of both surfaces of the first main body portion 21 with the second main body portions 22 and 23 having a film shape. The first main body portion 21 having the grooves and the through-holes can be relatively easily produced by injection molding or the like.

In addition, the first inlet portion flow path 61 is formed of the groove formed in the lower surface of the first main body portion 21 and an upper surface of the lower second main body portion 23 having hydrophilicity, and the first inlet portion flow path 61 has hydrophilicity at least in an upper surface portion of the lower second main body portion 23, namely, in a lower inner wall of the first inlet portion flow path 61. In addition, the second inlet portion flow path 62 is formed of the groove formed in the upper surface of the first main body portion 21 and a lower surface of the upper second main body portion 22 having hydrophobicity. A hydrophilic portion does not exist in the second inlet portion flow path 62.

Since the first inlet portion flow path 61 and the second inlet portion flow path 62 are configured as described above, when the primary dispensing of the liquid L is performed, the liquid L can evenly flow into a plurality of the first inlet portion flow paths 61. Meanwhile, since an inner wall of the second inlet portion flow path 62 has hydrophobicity and a barrier in a height direction exist in the connection portion between the first inlet portion flow path 61 and the second inlet portion flow path 62, the inflow of the liquid L to the second inlet portion flow path 62 is blocked. Incidentally, the first inlet portion flow path 61 may be formed on an upper side of the first main body portion 21 and the second inlet portion flow path 62 may be formed on a lower side of the main body portion 21, but in this case, since a barrier effect in the height direction cannot be obtained in the connection portion between both flow path portions, it is preferable that the first inlet portion flow path 61 and the second inlet portion flow path 62 are disposed on the lower side and the upper side, respectively.

In addition, since the reaction chamber portion 63 is formed by closing the through-hole, which is provided in the first main body portion 21, with the upper second main body portion 22 and the lower second main body portion 23, at least the upper surface of the lower second main body portion 23 has hydrophilicity. For this reason, when a positive pressure or negative pressure is applied to the dispensed liquid L staying in the first inlet portion flow path 61, the liquid L which has passed through the second inlet portion flow path 62 can easily flow into and stay in the reaction chamber portion 63 (secondary dispensing). Meanwhile, since a hydrophilic portion does not exist and a barrier in the height direction exists in the second inlet portion flow path 62 and the outlet portion side first flow path 71, the backflow or leakage of the liquid L is unlikely to occur. Incidentally, the outlet portion side first flow path 71 may be formed on the lower side of the main body portion 21, but in this case, since a barrier effect in the height direction cannot be obtained, it is preferable that the outlet portion side first flow path 71 is disposed on the upper side.

### (Effects of Second Embodiment)

According to the second embodiment described above, it is easier to produce the flow path device 10 as compared to that of the first embodiment. In the invention, it is necessary to provide a hydrophilic portion and a hydrophobic portion in each flow path. However, it is not easy to accurately apply a hydrophilic treatment or a hydrophobic treatment to only one portion of one member, and it can be easier to apply a treatment to one member as a whole. In the second embodiment, it is enough to apply a hydrophilic treatment to the entire upper surface of the lower second main body portion 23 having a film shape (and/or a hydrophobic treatment to the entire lower surface of the upper second main body portion 22), and the flow path device 10 can be relatively easily and reliably produced.

### [III] Modification Examples of Embodiments

The embodiments of the invention have been described above; however, the specific configurations and devices of the invention can be arbitrarily modified and improved without departing from the technical concept of each invention described in the claims. Hereinafter, such modification examples will be described.

### (Regarding Technical Problems or Effects of Invention)

First, technical problems or effects of the invention are not limited to the above contents, and problems which are not described above can be solved or effects which are not described above can be displayed by the invention, and only a part of the described problems can be solved or only a part of the described effects can be displayed.

### (Regarding Shape, Numerical Value, Structure, and Time Series)

Regarding the components illustrated in the embodiments or the drawings, the shapes, the numerical values, or the structure or an interrelationship of a plurality of the components can be arbitrarily modified and improved without departing from the technical concept of the invention.

### (Regarding Test System)

In the first and second embodiments, the test system 1 has been described as including the discharge and suction unit and the attachment and detachment unit; however, the invention is not limited thereto. For example, at least one of the discharge and suction unit and the attachment and detachment unit may be omitted. In this case, work for discharging or suctioning the liquid L or work for attaching or removing the lid portion 80 may be manually performed. Alternatively, the discharge and suction unit and the attachment and detachment unit may be integrally configured.

### (Regarding Flow Path Device)

In the first and second embodiments, the first main body portion 21, the upper second main body portion 22, and the lower second main body portion 23 have been described as being formed separately from each other; however, the invention is not limited thereto. For example, the first main body portion 21 may be integrally formed with one or both of the upper second main body portion 22 and the lower second main body portion 23 by a 3D printer or the like. Particularly, when the first main body portion 21 and the lower second main body portion 23 are integrally formed, the inlet portion side first flow paths 61, the inlet portion side second flow paths 62, and the reaction chamber portions 63 are formed in a groove shape in the first main body portion 21 and the lower second main body portion 23 which are integrally formed. In this case, the main body portion 20 can be created by adding real-time PCR reagents containing different primers to the reaction chamber portions 63 formed in a groove shape, for example, for the respective flow paths, and then attaching the upper second main body portion 22 to the first main body portion 21 and the lower second main body portion 23 by bonding, welding, or the like. In addition, when the first main body portion 21, the upper second main body portion 22, and the lower second main body portion 23 are integrally formed, for example, an injection portion (as one example, an injection portion including an opening portion or a flow path) through which a reagent can be injected into each of the reaction chamber portions 63 of the flow path device 10 from outside is provided in each of the reaction chamber portions 63, so that real-time PCR reagents containing different primers can be injected into the respective injection portions to create the flow path device 10 having the same configuration as the above configuration.

In addition, in the first and second embodiments, the main body portion 20 has been described as including the upper second main body portion 22 and the lower second main body portion 23; however, the invention is not limited thereto. For example, when an open surface of the flow path 50 is formed in only one of the upper surface and the lower surface of the first main body portion 21, one of the upper second main body portion 22 and the lower second main body portion 23 may be omitted.

In addition, in the first and second embodiments, the inlet portion 30 and the outlet portion 40 have been described as being concentrically disposed; however, the invention is not limited thereto, and the inlet portion 30 and the outlet portion 40 may be non-concentrically disposed. As one example, the inlet portion 30 and the outlet portion 40 may be disposed so as to partially overlap each other. Alternatively, the inlet portion 30 and the outlet portion 40 may be adjacently disposed so as not to overlap each other. The inlet portion 30 and the outlet portion 40 can be integrally closed with one lid portion 80 by concentrically or adjacently disposing the inlet portion 30 and the outlet portion 40, which is an advantage. However, the invention is not limited thereto. For example, the inlet portion 30 and the outlet portion 40 may be disposed apart from each other in the right-left direction or the front-rear direction of the flow path device 10.

In addition, in the first and second embodiments, the number of the inlet portions 30 which are installed has been described as being 1; however, the invention is not limited thereto, and the number of the inlet portions 30 which are installed may be, for example, 2 or more. In this case, for example, each of the inlet portion side first flow paths 61 may include a plurality of the first flow path side connection portions 61a connected to one of two or more inlet portions 30, and the first flow path side main body portion 61b connected to each of the plurality of first flow path side connection portions 61a.

In addition, in the first and second embodiments, the number of the outlet portions 40 which are installed has been described as being 1; however, the invention is not limited thereto, and the number of the outlet portions 40 which are installed may be, for example, 2 or more. In this case, for example, the first outlet portion side flow path 70a or the second outlet portion side flow path 70b may include a plurality of the outlet portion side fifth flow paths 75 connected to two or more outlet portions 40. Particularly, when a plurality of reactions are performed on one sample, it is preferable that one or a small number of the inlet portions 30 are provided, but since the outlet portion 40 is an air vent through air or the like in the flow path 50 is pushed out when the liquid L is injected into the flow path device 10, the outlet portions 40 of the same number as that of the reaction chamber portions 63 may be disposed apart from the inlet portions 30.

### (Regarding Flow Path)

In the first and second embodiments, the number of the inlet portion side first flow paths 61 has been described as being 3; however, the invention is not limited thereto, and the number of the inlet portion side first flow paths 61 may be, for example, 2 or 4 or more. In this case, the number of the inlet portion side second flow paths 62 or the reaction chamber portions 63 may be increased or decreased according to an increase or a decrease in number of the inlet portion side first flow paths 61.

In addition, in the first and second embodiments, the hydraulic equivalent diameters of the inlet portion side first flow paths 61 have been described as being set to be the same; however, the invention is not limited thereto. For example, when a positive pressure or negative pressure is applied to the liquid L in each of the inlet portion side first flow paths 61, in a case where the amounts of the liquid L flowing out from the inlet portion side first flow paths 61 toward the corresponding reaction chamber portions 63 are substantially the same, the hydraulic equivalent diameters of the inlet portion side first flow paths 61 may be different from each other. In addition, the lengths of the inlet portion side first flow paths 61 have been described as being set to be the same; however, the invention is not limited thereto. For example, when a positive pressure or negative pressure is applied to the liquid L in each of the inlet portion side first flow paths 61, in a case where the amounts of the liquid L flowing out from the inlet portion side first flow paths 61 toward the corresponding reaction chamber portions 63 are substantially the same, the lengths of the inlet portion side first flow paths 61 may be different from each other.

In addition, in the first and second embodiments, the size of each of the inlet portion side first flow paths 61 has been described as being set such that the containing capacity of each of the inlet portion side first flow paths 61 is substantially the same as the containing capacity of the corresponding reaction chamber portion 63; however, the invention is not limited thereto. For example, the size of each of the inlet portion side first flow paths 61 may be set such that the containing capacity of each of the inlet portion side first flow paths 61 is smaller than the containing capacity of the corresponding reaction chamber portion 63. As one example, the hydraulic equivalent diameter of each of the inlet portion side first flow paths 61 may be set to be smaller than the hydraulic equivalent diameter of each of the inlet portion side first flow paths 61 according to the first embodiment, or the length of each of the inlet portion side first flow paths 61 may be set to be shorter than the length of each of the inlet portion side first flow paths 61 according to the first embodiment. In this case, for example, in the injection step of the test method, the liquid L may be injected into the inlet portion side first flow path 61 of each of the first inlet portion side flow path 60a, the second inlet portion side flow path 60b, and the third inlet portion side flow path 60c via the inlet portion 30, and a positive pressure may be applied to the liquid L by causing the liquid L to be discharged from the nozzle of the discharge and suction unit via the pipette tip 11.

### (Regarding Lid Portion)

In the first and second embodiments, the lid portion 80 has been described as being formed as a columnar body made of resin; however, the invention is not limited thereto, and the lid portion 80 may be formed as, for example, a flat thin film-shaped body (as one example, a seal made of resin, or the like). In addition, when the inlet portion 30 and the outlet portion 40 are disposed apart from each other (a plurality of the inlet portions 30 and the outlet portions 40 may be disposed), the lid portion 80 may be shaped to close the inlet portion 30 and the outlet portion 40 one by one, or may be shaped to collectively close the inlet portion 30 and the outlet portion 40. As one example of the configuration of the lid portion 80, there is a film-shaped seal or the like that covers the entire upper surface of the upper second main body portion 22. As the material of the lid portion 80 in this case, for example, polypropylene or the like can be suitably used.

### (Regarding Test Method)

In the first and second embodiments, the test method has been described as including the injection step, the amplification step, and the detection step; however, the invention is not limited thereto. For example, when the flow path device 10 is not assembled or when a real-time PCR reagent is not contained in the reaction chamber portion 63, a preparation step of performing the work may be further added. In addition, the reagent to be contained is not limited to the real-time PCR reagents, and may be reagents for biochemical tests, hematology tests, immunological tests, or other nucleic acid tests.

In addition, in the first and second embodiments, the configuration has been described in which in the injection step, a positive pressure is applied to the liquid L in each of the inlet portion side first flow paths 61 by causing air to be discharged from the nozzle of the discharge and suction unit via the pipette tip 11; however, the invention is not limited thereto. For example, a suction opening portion connected to each of the reaction chamber portions 63 via a predetermined flow path may be provided in the flow path device 10, and a negative pressure may be applied to the liquid L in each of the inlet portion side first flow paths 61 by causing air to be suctioned from a nozzle of a suction unit not illustrated via the suction opening portion. In this case, the magnitude of the negative pressure applied to the liquid L in each of the inlet portion side first flow paths 61 is the threshold value or greater, so that the liquid L flows out from each of the inlet portion side first flow paths 61 to the corresponding reaction chamber portion 63, and the application of the negative pressure may continue to be performed until all the liquid L in each of the inlet portion side first flow paths 61 flows out.

### (Notes)

A biological component test flow path device of note 1 is a flow path device that contains a liquid containing a test object to be used for a biochemical test, a hematology test, a nucleic acid test, or an immunological test, the device comprising: a main body portion; a flow path provided inside the main body portion; an inlet portion which is provided so as to be exposed to an outside of the main body portion and is connected to the flow path, and through which the liquid flows into the flow path; and an outlet portion which is provided so as to be exposed to the outside of the main body portion and is connected to the flow path, and through which air in the flow path flows out to the outside, wherein the flow path includes a plurality of first flow paths connected to the inlet portion and having hydrophilicity, a plurality of second flow paths each of which is connected to one of the plurality of first flow paths, and a plurality of reaction chamber portions each of which is used for reaction of the liquid and is connected to one of the plurality of second flow paths, the plurality of first flow paths are configured such that when a positive pressure or negative pressure is applied to the liquid in the plurality of first flow paths by a predetermined method, amounts of the liquid flowing out from the plurality of first flow paths toward the corresponding reaction chamber portions are substantially the same, and the plurality of second flow paths are configured such that when a magnitude of the positive pressure or negative pressure applied to the liquid in the plurality of first flow paths is less than a threshold value, an outflow of the liquid from the first flow path, which is connected to each of the plurality of second flow paths, to the corresponding reaction chamber portion is restricted, and when the magnitude of the applied positive pressure or negative pressure is the threshold value or greater, an outflow of the liquid is allowed.

The flow path device of note 2 according to the flow path device of note 1, wherein the inlet portion is a dispensing opening through which the liquid is dispensed to the plurality of first flow paths, and is an application opening through which a positive pressure is applied to the liquid in the plurality of first flow paths.

The flow path device of note 3 according to the flow path device of note 1 or 2, wherein hydraulic equivalent diameters of the plurality of first flow paths are set to be the same, and lengths of the plurality of first flow paths are set to be the same.

The flow path device of note 4 according to the flow path device of any one of notes 1 to 3, wherein the plurality of first flow paths are formed in a meandering shape.

The flow path device of note 5 according to the flow path device of any one of notes 1 to 4, wherein at least a part of each of inner walls of the plurality of first flow paths has hydrophilicity over an entire length of the first flow path.

The flow path device of note 6 according to the flow path device of any one of notes 1 to 5, wherein the number of the reaction chamber portions connected to each of the plurality of second flow paths is set to a power of 2.

The flow path device of note 7 according to the flow path device of any one of notes 1 to 6, wherein in each of inner walls of the plurality of second flow paths, at least a portion connected to the first flow path and a portion connected to the reaction chamber portion have hydrophobicity.

The flow path device of note 8 according to the flow path device of any one of notes 1 to 7, wherein at least a part of each of inner walls of the plurality of reaction chamber portions has hydrophilicity.

A test system of note 9 comprising: the flow path device according to any one of notes 1 to 8.

### (Effect of notes)

According to the flow path device of note 1 and the test system of note 9, since the flow path includes a plurality of first flow paths connected to the inlet portion and having hydrophilicity, a plurality of second flow paths each of which is connected to one of the plurality of first flow paths, and a plurality of reaction chamber portions each of which is used for reaction of the liquid and is connected to one of the plurality of second flow paths, the plurality of first flow paths are configured such that when a positive pressure or negative pressure is applied to the liquid in the plurality of first flow paths by a predetermined method, amounts of the liquid flowing out from the plurality of first flow paths toward the corresponding reaction chamber portions are substantially the same, and the plurality of second flow paths are configured such that when a magnitude of the positive pressure or negative pressure applied to the liquid in the plurality of first flow paths is less than a threshold value, an outflow of the liquid from the first flow path, which is connected to each of the plurality of second flow paths, to the corresponding reaction chamber portion is restricted, and when the magnitude of the applied positive pressure or negative pressure is the threshold value or greater, an outflow of the liquid is allowed, when the liquid is primary dispensed to the plurality of first flow paths via the inlet portion, and a positive pressure or negative pressure is applied to the primary dispensed liquid, the liquid can secondary flow out to the plurality of reaction chamber portions, and the labor of work for dispensing the liquid can be further reduced as compared to the technique of the related art (technique in which the supply port and the discharge port are provided in each of the plurality of flow paths). Particularly, since the plurality of first flow paths can be configured such that the amounts of the liquid flowing out from the plurality of first flow paths are substantially the same, the liquid can evenly flow out to the plurality of reaction chamber portions, and quantitative dispensing to the plurality of reaction chamber portions can be realized. In addition, since the plurality of second flow paths can be configured such that when the magnitude of the positive pressure or negative pressure applied to the liquid in the plurality of first flow paths is less than the threshold value, the outflow of the liquid is restricted, and when the magnitude of the applied positive pressure or negative pressure is the threshold value or greater, the outflow of the liquid is allowed, regardless of the number of the first flow paths that are installed, quantitative dispensing to the plurality of reaction chamber portions can be reliably performed. For the above reasons, the ease of use of the flow path device can be improved.

According to the flow path device of note 2, since the inlet portion is a dispensing opening through which the liquid is dispensed to the plurality of first flow paths, and is an application opening through which a positive pressure is applied to the liquid in the plurality of first flow paths, the dispensing of the liquid or the application of a positive pressure can be performed via the inlet portion, and the labor of work for dispensing the liquid and work for applying a positive pressure can be further reduced as compared to when the dispensing opening and the application opening are individually provided.

According to the flow path device of note 3, since hydraulic equivalent diameters of the plurality of first flow paths are set to be the same, and lengths of the plurality of first flow paths are set to be the same, the amounts of the liquid flowing out from the plurality of first flow paths can be made substantially the same, and when positive pressures or negative pressures are applied to the liquid in the plurality of first flow paths, the magnitudes of the applied positive pressures or negative pressures can be made substantially the same, so that quantitative dispensing to the plurality of reaction chamber portions is easily realized.

According to the flow path device of note 4, since the plurality of first flow paths are formed in a meandering shape, it is possible to make the first flow paths compact and increase the containing capacity, and the ease of use of the flow path device is easily improved.

According to the flow path device of note 5, since at least a part of each of inner walls of the plurality of first flow paths has hydrophilicity over an entire length of the first flow path, the plurality of first flow paths can reliably have hydrophilicity, and the function of the plurality of first flow paths can be reliably exhibited.

According to the flow path device of note 6, since the number of the reaction chamber portions connected to each of the plurality of second flow paths is set to a power of 2, the liquid can evenly flow into the reaction chamber portions connected to each of the plurality of second flow paths, and quantitative dispensing to the plurality of reaction chamber portions is easily realized.

According to the flow path device of note 7, since in each of inner walls of the plurality of second flow paths, at least a portion connected to the first flow path and a portion connected to the reaction chamber portion have hydrophobicity, the plurality of second flow paths can have hydrophobicity, and unnecessary inflow of the liquid from the first flow path to the second flow paths and the backflow of the liquid from the reaction chamber portions to the second flow paths can be reliably restricted.

According to the flow path device of note 8, since at least a part of each of inner walls of the plurality of reaction chamber portions has hydrophilicity, the plurality of reaction chamber portions can have hydrophilicity. Therefore, since the reaction chamber portion is a place in which a desired reaction is performed on the liquid which is a sample, and the liquid is normally an aqueous medium, the liquid can flow into the reaction chamber portion and the desired reaction can be performed.

### Reference Signs List

- 1: Test system
- 10: Flow path device
- 11: Pipette tip
- 20: Main body portion
- 21: First main body portion
- 22: Upper second main body portion
- 23: Lower second main body portion
- 30: Inlet portion
- 40: Outlet portion
- 50: Flow path
- 60: Inlet portion side flow path
- 60a: First inlet portion side flow path
- 60b: Second inlet portion side flow path
- 60c: Third inlet portion side flow path
- 61: Inlet portion side first flow path
- 61a: First flow path side connection portion
- 61b: First flow path side main body portion
- 62: Inlet portion side second flow path
- 62a: Second flow path side first connection portion
- 62b: Second flow path side second connection portion
- 63, 63a, 63b: Reaction chamber portion
- 70: Outlet portion side flow path
- 70a: First outlet portion side flow path
- 70b: Second outlet portion side flow path
- 71, 71a, 71b, 71c: Outlet portion side first flow path
- 72: Outlet portion side second flow path
- 73: Outlet portion side third flow path
- 74: Outlet portion side fourth flow path
- 75: Outlet portion side fifth flow path
- 76: Outlet portion side sixth flow path
- 80: Lid portion
- 90: Temperature regulating unit
- L: Liquid

## Claims

1. A flow path device that contains a liquid containing a test object to be used for a biochemical test, a hematology test, a nucleic acid test, or an immunological test, the device comprising:
a main body portion;
a flow path provided inside the main body portion;
an inlet portion which is provided so as to be exposed to an outside of the main body portion and is connected to the flow path, and through which the liquid flows into the flow path; and
an outlet portion which is provided so as to be exposed to the outside of the main body portion and is connected to the flow path, and through which air in the flow path flows out to the outside,
wherein the flow path includes a plurality of first flow paths connected to the inlet portion and having hydrophilicity, a plurality of second flow paths each of which is connected to one of the plurality of first flow paths, and a plurality of reaction chamber portions each of which is used for reaction of the liquid and is connected to one of the plurality of second flow paths,
the plurality of first flow paths are configured such that when a positive pressure or negative pressure is applied to the liquid in the plurality of first flow paths by a predetermined method, amounts of the liquid flowing out from the plurality of first flow paths toward the corresponding reaction chamber portions are substantially the same, and
the plurality of second flow paths are configured such that when a magnitude of the positive pressure or negative pressure applied to the liquid in the plurality of first flow paths is less than a threshold value, an outflow of the liquid from the first flow path, which is connected to each of the plurality of second flow paths, to the corresponding reaction chamber portion is restricted, and when the magnitude of the applied positive pressure or negative pressure is the threshold value or greater, an outflow of the liquid is allowed.

2. The flow path device according to claim 1,
wherein the inlet portion is a dispensing opening through which the liquid is dispensed to the plurality of first flow paths, and is an application opening through which a positive pressure is applied to the liquid in the plurality of first flow paths.

3. The flow path device according to claim 1 or 2,
wherein hydraulic equivalent diameters of the plurality of first flow paths are set to be the same, and
lengths of the plurality of first flow paths are set to be the same.

4. The flow path device according to any one of claims 1 to 3,
wherein the plurality of first flow paths are formed in a meandering shape.

5. The flow path device according to any one of claims 1 to 4,
wherein at least a part of each of inner walls of the plurality of first flow paths has hydrophilicity over an entire length of the first flow path.

6. The flow path device according to any one of claims 1 to 5,
wherein the number of the reaction chamber portions connected to each of the plurality of second flow paths is set to a power of 2.

7. The flow path device according to any one of claims 1 to 6,
wherein in each of inner walls of the plurality of second flow paths, at least a portion connected to the first flow path and a portion connected to the reaction chamber portion have hydrophobicity.

8. The flow path device according to any one of claims 1 to 7,
wherein at least a part of each of inner walls of the plurality of reaction chamber portions has hydrophilicity.

9. A test system comprising:
the flow path device according to any one of claims 1 to 8.
